# EUROPEAN PATENT APPLICATION

(11) **EP 3 521 299 A1**
(43) Date of publication of application: **07.08.2019**
(21) Application number: 17858396.9
(22) Date of filing: 03.10.2017
(51) Int. Cl.: C07K 1/14, C07K 1/30, C07K 1/36, C07K 14/435, C12P 21/02, D01F 6/68

(54) **METHOD FOR PURIFYING RECOMBINANT PROTEIN**

(30) Priority: 03.10.2016 JP 2016195644
(71) Applicant: Spiber Inc., Yamagata 997-0052 (JP)
(72) Inventor: HOMMA, Toshimasa, Tsuruoka-shi Yamagata 997-0052 (JP)
(74) Representative: Handley, Matthew Edward
(86) International application number: PCT/JP2017/035983
(87) International publication number: WO 2018/066558

(57) **Abstract**

Disclosed is a method for purifying a recombinant protein of interest from a recombinant cell expressing intracellularly the recombinant protein as an insoluble matter, comprising treating the recombinant cell under conditions where a protein derived from a host cell dissolves in a first aprotic polar solvent to which an inorganic salt is added or not but the recombinant protein does not dissolve, removing a first soluble fraction, and then treating the resulting first insoluble fraction under conditions where the recombinant protein dissolves in a second aprotic polar solvent to which an inorganic salt is added.

SEQUENCE LISTING

<110>  Spiber Inc.
       KOJIMA INDUSTRIES CORPORATION

<120>  A method for puryfing an insoluble recombinant protein

<130>  FP17-0540-00

<160>  8

<170>  PatentIn version 3.5

<210>  1
<211>  576
<212>  PRT
<213>  Artificial Sequence

<220>
<223>  PRT468

<400>  1

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Gly Pro Gly
65                  70                  75                  80

Gln Gln Gly Pro Ser Ala Ser Ala Ala Ala Ala Ala Ala Ala Gly Pro
                85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala
        115                 120                 125

Ala Ala Ala Gly Ser Tyr Gly Ser Gly Pro Gly Gln Gln Gly Pro Tyr
    130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Gln Gln Gly Pro Ser Ala Ser Ala Ala Ala Ala Ala Ala Ala
            180                 185                 190

Gly Ser Gly Gln Gln Gly Pro Gly Gln Tyr Gly Pro Tyr Ala Ser Ala
        195                 200                 205

Ala Ala Ala Ala Ala Ala Gly Ser Tyr Gly Ser Gly Pro Gly Gln Gln
    210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Ala Gly Pro
                245                 250                 255

Gly Gln Gln Gly Pro Tyr Gly Pro Gly Ser Ser Ala Ala Ala Ala Ala
            260                 265                 270

Ala Ala Gly Ser Tyr Gly Tyr Gly Pro Gly Gln Gln Gly Pro Tyr Gly
        275                 280                 285

Pro Gly Ala Ser Gly Gln Asn Gly Pro Gly Ser Gly Gln Tyr Gly Pro
    290                 295                 300

Gly Gln Gln Gly Pro Gly Pro Ser Ala Ala Ala Ala Ala Ala Ala Gly
305                 310                 315                 320

Pro Gly Gln Gln Gly Pro Tyr Gly Pro Gly Ala Ser Ala Ala Ala Ala
                325                 330                 335

Ala Ala Ala Gly Ser Tyr Gly Pro Gly Gln Gln Gly Pro Gly Gln Tyr
            340                 345                 350

Gly Pro Gly Ser Ser Gly Pro Gly Gln Gln Gly Pro Tyr Gly Pro Gly
        355                 360                 365

Ser Ser Ala Ala Ala Ala Ala Ala Ala Gly Ser Tyr Gly Pro Gly Gln
    370                 375                 380

Gln Gly Pro Tyr Gly Pro Gly Pro Ser Ala Ala Ala Ala Ala Ala Ala
385                 390                 395                 400

Gly Ser Tyr Gln Gln Gly Pro Gly Gln Gln Gly Pro Tyr Gly Pro Gly
                405                 410                 415

Ala Ser Gly Pro Gly Gln Gln Gly Pro Tyr Gly Pro Gly Ala Ser Ala
                420                 425                 430

Ala Ala Ala Ala Ala Ala Gly Pro Gly Gln Tyr Gly Pro Gly Gln Gln
        435                 440                 445

Gly Pro Ser Ala Ser Ala Ala Ala Ala Ala Ala Ala Gly Ser Tyr Gly
    450                 455                 460

Ser Gly Pro Gly Gln Tyr Gly Pro Tyr Gly Pro Gly Gln Ser Gly Pro
465                 470                 475                 480

Gly Ser Gly Gln Gln Gly Gln Gly Pro Tyr Gly Pro Gly Ala Ser Ala
                485                 490                 495

Ala Ala Ala Ala Ala Ala Gly Ser Tyr Gly Pro Gly Gln Gln Gly Pro
                500                 505                 510

Tyr Gly Pro Gly Pro Ser Ala Ala Ala Ala Ala Ala Ala Gly Pro Gly
        515                 520                 525

Ser Gly Gln Tyr Gly Pro Gly Ala Ser Gly Gln Asn Gly Pro Gly Ser
    530                 535                 540

Gly Gln Tyr Gly Pro Gly Gln Gln Gly Pro Gly Pro Ser Ala Ala Ala
545                 550                 555                 560

Ala Ala Ala Ala Gly Pro Gly Ser Gly Gln Gln Gly Pro Gly Ala Ser
                565                 570                 575

<210>  2
<211>  595
<212>  PRT
<213>  Artificial Sequence

<220>
<223>  PRT647

<400>  2

Met His His His His His His Ser Ser Gly Ser Ser Thr Thr Met Asn
1               5                   10                  15

Trp Ser Thr Arg Leu Val Leu Ser Ile Leu Val Val Leu Cys Thr Gln
            20                  25                  30

Ser Leu Cys Ala Leu Gly Gln Ala Asn Thr Pro Trp Ser Ser Lys Glu
        35                  40                  45

Asn Ala Asp Ala Phe Ile Gly Ala Phe Met Asn Ala Ala Ser Gln Ser
    50                  55                  60

Gly Ala Phe Ser Ser Asp Gln Ile Asp Asp Met Ser Val Ile Ser Asn
65                  70                  75                  80

Thr Leu Met Ala Ala Met Asp Asn Met Gly Gly Arg Ile Thr Gln Ser
                85                  90                  95

Lys Leu Gln Ala Leu Asp Met Ala Phe Ala Ser Ser Val Ala Glu Ile
            100                 105                 110

Ala Val Ala Asp Gly Gln Asn Val Gly Ala Ala Thr Asn Ala Ile Ser
        115                 120                 125

Asp Ala Leu Arg Ser Ala Phe Tyr Gln Thr Thr Gly Val Val Asn Asn
    130                 135                 140

Gln Phe Ile Thr Gly Ile Ser Ser Leu Ile Gly Met Phe Ala Gln Val
145                 150                 155                 160

Ser Gly Asn Glu Val Ser Tyr Ser Ser Ala Gly Ser Ser Ser Ala Ala
                165                 170                 175

Ala Ser Glu Ala Val Ser Ala Gly Gln Gly Pro Ala Ala Gln Pro Val
            180                 185                 190

Tyr Ala Pro Ser Ala Ser Ala Ala Ala Ala Ala Ala Ser Gly Ala Ala
        195                 200                 205

Pro Ala Ile Gln Gln Ala Tyr Glu Arg Gly Gly Ser Gly Ser Ala Ala
    210                 215                 220

Ala Ala Ala Gly Ser Gly Pro Ser Gly Tyr Gly Gln Gly Ala Gly Gly
225                 230                 235                 240

Pro Gly Gly Ala Gly Ala Ala Ala Gly Ala Ala Ala Ala Gly Gly Ser
                245                 250                 255

Gly Pro Gly Gly Tyr Gly Gln Gly Pro Ala Ala Tyr Gly Pro Ser Gly
            260                 265                 270

Pro Ser Gly Gln Gln Gly Tyr Gly Pro Gly Gly Ser Gly Ala Ala Ala
        275                 280                 285

Ala Ala Ala Ala Ala Ala Gly Ser Gly Pro Ser Gly Tyr Gly Pro Gly
    290                 295                 300

Ala Gly Gly Pro Gly Gly Ala Gly Ala Ala Ala Ala Ala Ala Ala Ala
305                 310                 315                 320

Gly Gly Ser Gly Pro Gly Gly Tyr Gly Gln Gly Gln Ala Ser Tyr Gly
                325                 330                 335

Pro Ser Gly Pro Ser Gly Gln Gln Gly Tyr Gly Pro Gly Gly Ser Gly
            340                 345                 350

Ala Ala Ala Ala Ala Ala Ala Ala Ala Gly Gly Pro Gly Phe Gly Gly
        355                 360                 365

Gln Gln Gly Tyr Gly Pro Gly Gly Ser Gly Ala Ala Ala Ala Ala Ala
    370                 375                 380

Ala Gly Gly Ala Gly Pro Gly Arg Gln Gln Ala Tyr Gly Pro Gly Gly
385                 390                 395                 400

Ser Gly Ala Ala Ala Ala Ala Ala Ala Ala Ala Gly Ser Gly Pro Ser
                405                 410                 415

Gly Tyr Gly Pro Ser Ala Ala Gly Pro Ser Gly Pro Gly Gly Ser Gly
            420                 425                 430

Ala Ala Gly Gly Ser Gly Pro Gly Gly Phe Gly Gln Gly Pro Ala Gly
        435                 440                 445

Tyr Gly Pro Ser Gly Pro Gly Gly Gln Gln Gly Tyr Gly Pro Gly Ala
    450                 455                 460

Ser Gly Ala Ala Ala Ala Ala Ala Ala Ser Gly Ser Gly Gly Tyr Gly
465                 470                 475                 480

Pro Ser Gln Tyr Val Pro Ser Ser Val Ala Ser Ser Ala Ala Ser Ala
                485                 490                 495

Ala Ser Ala Leu Ser Ser Pro Thr Thr His Ala Arg Ile Ser Ser His
            500                 505                 510

Ala Ser Thr Leu Leu Ser Ser Gly Pro Thr Asn Ala Ala Ala Leu Ser
        515                 520                 525

Asn Val Ile Ser Asn Ala Val Ser Gln Val Ser Ala Ser Asn Pro Gly
    530                 535                 540

Ser Ser Ser Cys Asp Val Leu Val Gln Ala Leu Leu Glu Ile Ile Thr
545                 550                 555                 560

Ala Leu Ile Ser Ile Leu Asp Ser Ser Ser Val Gly Gln Val Asn Tyr
                565                 570                 575

Gly Ser Ser Gly Gln Tyr Ala Gln Ile Val Gly Gln Ser Met Gln Gln
            580                 585                 590

Ala Met Gly
        595

<210>  3
<211>  576
<212>  PRT
<213>  Artificial Sequence

<220>
<223>  PRT699

<400>  3

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Gln Ser Gly
        35                  40                  45

Gln Tyr Gly Pro Gly Val Leu Gly Pro Gly Val Leu Gly Pro Gly Ser
    50                  55                  60

Ser Ala Ala Ala Ala Ala Ala Ala Gly Pro Gly Gln Tyr Gly Pro Gly
65                  70                  75                  80

Val Leu Gly Pro Ser Ala Ser Ala Ala Ala Ala Ala Ala Ala Gly Pro
                85                  90                  95

Gly Ser Gly Val Leu Gly Pro Gly Ala Ser Gly Gln Tyr Gly Pro Gly
            100                 105                 110

Val Leu Gly Pro Gly Val Leu Gly Pro Gly Ser Ser Ala Ala Ala Ala
        115                 120                 125

Ala Ala Ala Gly Ser Tyr Gly Ser Gly Pro Gly Val Leu Gly Pro Tyr
    130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Val Leu Gly Pro Ser Ala Ser Ala Ala Ala Ala Ala Ala Ala
            180                 185                 190

Gly Ser Gly Val Leu Gly Pro Gly Gln Tyr Gly Pro Tyr Ala Ser Ala
        195                 200                 205

Ala Ala Ala Ala Ala Ala Gly Ser Tyr Gly Ser Gly Pro Gly Val Leu
    210                 215                 220

Gly Pro Tyr Gly Pro Gly Gln Ser Gly Ser Gly Val Leu Gly Pro Gly
225                 230                 235                 240

Val Leu Gly Pro Tyr Ala Ser Ala Ala Ala Ala Ala Ala Ala Gly Pro
                245                 250                 255

Gly Val Leu Gly Pro Tyr Gly Pro Gly Ser Ser Ala Ala Ala Ala Ala
            260                 265                 270

Ala Ala Gly Ser Tyr Gly Tyr Gly Pro Gly Val Leu Gly Pro Tyr Gly
        275                 280                 285

Pro Gly Ala Ser Gly Gln Asn Gly Pro Gly Ser Gly Gln Tyr Gly Pro
    290                 295                 300

Gly Val Leu Gly Pro Gly Pro Ser Ala Ala Ala Ala Ala Ala Ala Gly
305                 310                 315                 320

Pro Gly Val Leu Gly Pro Tyr Gly Pro Gly Ala Ser Ala Ala Ala Ala
                325                 330                 335

Ala Ala Ala Gly Ser Tyr Gly Pro Gly Val Leu Gly Pro Gly Gln Tyr
            340                 345                 350

Gly Pro Gly Ser Ser Gly Pro Gly Val Leu Gly Pro Tyr Gly Pro Gly
        355                 360                 365

Ser Ser Ala Ala Ala Ala Ala Ala Ala Gly Ser Tyr Gly Pro Gly Val
    370                 375                 380

Leu Gly Pro Tyr Gly Pro Gly Pro Ser Ala Ala Ala Ala Ala Ala Ala
385                 390                 395                 400

Gly Ser Tyr Val Leu Gly Pro Gly Val Leu Gly Pro Tyr Gly Pro Gly
                405                 410                 415

Ala Ser Gly Pro Gly Val Leu Gly Pro Tyr Gly Pro Gly Ala Ser Ala
            420                 425                 430

Ala Ala Ala Ala Ala Ala Gly Pro Gly Gln Tyr Gly Pro Gly Val Leu
        435                 440                 445

Gly Pro Ser Ala Ser Ala Ala Ala Ala Ala Ala Ala Gly Ser Tyr Gly
    450                 455                 460

Ser Gly Pro Gly Gln Tyr Gly Pro Tyr Gly Pro Gly Gln Ser Gly Pro
465                 470                 475                 480

Gly Ser Gly Val Leu Gly Gln Gly Pro Tyr Gly Pro Gly Ala Ser Ala
                485                 490                 495

Ala Ala Ala Ala Ala Ala Gly Ser Tyr Gly Pro Gly Val Leu Gly Pro
            500                 505                 510

Tyr Gly Pro Gly Pro Ser Ala Ala Ala Ala Ala Ala Ala Gly Pro Gly
        515                 520                 525

Ser Gly Gln Tyr Gly Pro Gly Ala Ser Gly Gln Asn Gly Pro Gly Ser
    530                 535                 540

Gly Gln Tyr Gly Pro Gly Val Leu Gly Pro Gly Pro Ser Ala Ala Ala
545                 550                 555                 560

Ala Ala Ala Ala Gly Pro Gly Ser Gly Val Leu Gly Pro Gly Ala Ser
                565                 570                 575

<210>  4
<211>  576
<212>  PRT
<213>  Artificial Sequence

<220>
<223>  PRT698

<400>  4

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Ile Ser Gly
        35                  40                  45

Ile Tyr Gly Pro Gly Val Leu Gly Pro Gly Val Leu Gly Pro Gly Ser
    50                  55                  60

Ser Ala Ala Ala Ala Ala Ala Ala Gly Pro Gly Ile Tyr Gly Pro Gly
65                  70                  75                  80

Val Leu Gly Pro Ser Ala Ser Ala Ala Ala Ala Ala Ala Ala Gly Pro
                85                  90                  95

Gly Ser Gly Val Leu Gly Pro Gly Ala Ser Gly Ile Tyr Gly Pro Gly
            100                 105                 110

Val Leu Gly Pro Gly Val Leu Gly Pro Gly Ser Ser Ala Ala Ala Ala
        115                 120                 125

Ala Ala Ala Gly Ser Tyr Gly Ser Gly Pro Gly Val Leu Gly Pro Tyr
    130                 135                 140

Gly Ser Ala Ala Ala Ala Ala Ala Ala Gly Pro Gly Ser Gly Ile Tyr
145                 150                 155                 160

Gly Ile Gly Pro Tyr Gly Pro Gly Ala Ser Gly Pro Gly Ile Tyr Gly
                165                 170                 175

Pro Gly Val Leu Gly Pro Ser Ala Ser Ala Ala Ala Ala Ala Ala Ala
            180                 185                 190

Gly Ser Gly Val Leu Gly Pro Gly Ile Tyr Gly Pro Tyr Ala Ser Ala
        195                 200                 205

Ala Ala Ala Ala Ala Ala Gly Ser Tyr Gly Ser Gly Pro Gly Val Leu
    210                 215                 220

Gly Pro Tyr Gly Pro Gly Ile Ser Gly Ser Gly Val Leu Gly Pro Gly
225                 230                 235                 240

Val Leu Gly Pro Tyr Ala Ser Ala Ala Ala Ala Ala Ala Ala Gly Pro
                245                 250                 255

Gly Val Leu Gly Pro Tyr Gly Pro Gly Ser Ser Ala Ala Ala Ala Ala
            260                 265                 270

Ala Ala Gly Ser Tyr Gly Tyr Gly Pro Gly Val Leu Gly Pro Tyr Gly
        275                 280                 285

Pro Gly Ala Ser Gly Ile Asn Gly Pro Gly Ser Gly Ile Tyr Gly Pro
    290                 295                 300

Gly Val Leu Gly Pro Gly Pro Ser Ala Ala Ala Ala Ala Ala Ala Gly
305                 310                 315                 320

Pro Gly Val Leu Gly Pro Tyr Gly Pro Gly Ala Ser Ala Ala Ala Ala
                325                 330                 335

Ala Ala Ala Gly Ser Tyr Gly Pro Gly Val Leu Gly Pro Gly Ile Tyr
            340                 345                 350

Gly Pro Gly Ser Ser Gly Pro Gly Val Leu Gly Pro Tyr Gly Pro Gly
        355                 360                 365

Ser Ser Ala Ala Ala Ala Ala Ala Ala Gly Ser Tyr Gly Pro Gly Val
    370                 375                 380

Leu Gly Pro Tyr Gly Pro Gly Pro Ser Ala Ala Ala Ala Ala Ala Ala
385                 390                 395                 400

Gly Ser Tyr Val Leu Gly Pro Gly Val Leu Gly Pro Tyr Gly Pro Gly
                405                 410                 415

Ala Ser Gly Pro Gly Val Leu Gly Pro Tyr Gly Pro Gly Ala Ser Ala
            420                 425                 430

Ala Ala Ala Ala Ala Ala Gly Pro Gly Ile Tyr Gly Pro Gly Val Leu
        435                 440                 445

Gly Pro Ser Ala Ser Ala Ala Ala Ala Ala Ala Ala Gly Ser Tyr Gly
    450                 455                 460

Ser Gly Pro Gly Ile Tyr Gly Pro Tyr Gly Pro Gly Ile Ser Gly Pro
465                 470                 475                 480

Gly Ser Gly Val Leu Gly Ile Gly Pro Tyr Gly Pro Gly Ala Ser Ala
                485                 490                 495

Ala Ala Ala Ala Ala Ala Gly Ser Tyr Gly Pro Gly Val Leu Gly Pro
            500                 505                 510

Tyr Gly Pro Gly Pro Ser Ala Ala Ala Ala Ala Ala Ala Gly Pro Gly
        515                 520                 525

Ser Gly Ile Tyr Gly Pro Gly Ala Ser Gly Ile Asn Gly Pro Gly Ser
    530                 535                 540

Gly Ile Tyr Gly Pro Gly Val Leu Gly Pro Gly Pro Ser Ala Ala Ala

545                 550                 555                 560

Ala Ala Ala Ala Gly Pro Gly Ser Gly Val Leu Gly Pro Gly Ala Ser
                565                 570                 575

<210>  5
<211>  252
<212>  PRT
<213>  Artificial Sequence

<220>
<223>  Collagen-type4-Kai

<400>  5

Met His His His His His His Ser Ser Gly Ser Ser Lys Asp Gly Val
1               5                   10                  15

Pro Gly Phe Pro Gly Ser Glu Gly Val Lys Gly Asn Arg Gly Phe Pro
            20                  25                  30

Gly Leu Met Gly Glu Asp Gly Ile Lys Gly Gln Lys Gly Asp Ile Gly
        35                  40                  45

Pro Pro Gly Phe Arg Gly Pro Thr Glu Tyr Tyr Asp Thr Tyr Gln Glu
    50                  55                  60

Lys Gly Asp Glu Gly Thr Pro Gly Pro Pro Gly Pro Arg Gly Ala Arg
65                  70                  75                  80

Gly Pro Gln Gly Pro Ser Gly Pro Pro Gly Val Pro Gly Ser Pro Gly
                85                  90                  95

Ser Ser Arg Pro Gly Leu Arg Gly Ala Pro Gly Trp Pro Gly Leu Lys
            100                 105                 110

Gly Ser Lys Gly Glu Arg Gly Arg Pro Gly Lys Asp Ala Met Gly Thr
        115                 120                 125

Pro Gly Ser Pro Gly Cys Ala Gly Ser Pro Gly Leu Pro Gly Ser Pro
    130                 135                 140

Gly Pro Pro Gly Pro Pro Gly Asp Ile Val Phe Arg Lys Gly Pro Pro
145                 150                 155                 160

Gly Asp His Gly Leu Pro Gly Tyr Leu Gly Ser Pro Gly Ile Pro Gly
                165                 170                 175

Val Asp Gly Pro Lys Gly Glu Pro Gly Leu Leu Cys Thr Gln Cys Pro
            180                 185                 190

Tyr Ile Pro Gly Pro Pro Gly Leu Pro Gly Leu Pro Gly Leu His Gly
        195                 200                 205

Val Lys Gly Ile Pro Gly Arg Gln Gly Ala Ala Gly Leu Lys Gly Ser
    210                 215                 220

Pro Gly Ser Pro Gly Asn Thr Gly Leu Pro Gly Phe Pro Gly Phe Pro
225                 230                 235                 240

Gly Ala Gln Gly Asp Pro Gly Leu Lys Gly Glu Lys
                245                 250

<210>  6
<211>  315
<212>  PRT
<213>  Artificial Sequence

<220>
<223>  Resilin-Kai

<400>  6

Met His His His His His His Ser Ser Gly Ser Ser Pro Glu Pro Pro
1               5                   10                  15

Val Asn Ser Tyr Leu Pro Pro Ser Asp Ser Tyr Gly Ala Pro Gly Gln
            20                  25                  30

Ser Gly Pro Gly Gly Arg Pro Ser Asp Ser Tyr Gly Ala Pro Gly Gly
        35                  40                  45

Gly Asn Gly Gly Arg Pro Ser Asp Ser Tyr Gly Ala Pro Gly Gln Gly
    50                  55                  60

Gln Gly Gln Gly Gln Gly Gln Gly Gly Tyr Ala Gly Lys Pro Ser Asp
65                  70                  75                  80

Ser Tyr Gly Ala Pro Gly Gly Gly Asp Gly Asn Gly Gly Arg Pro Ser
                85                  90                  95

Ser Ser Tyr Gly Ala Pro Gly Gly Gly Asn Gly Gly Arg Pro Ser Asp
            100                 105                 110

Thr Tyr Gly Ala Pro Gly Gly Gly Asn Gly Gly Arg Pro Ser Asp Thr
        115                 120                 125

Tyr Gly Ala Pro Gly Gly Gly Gly Asn Gly Asn Gly Gly Arg Pro Ser
    130                 135                 140

Ser Ser Tyr Gly Ala Pro Gly Gln Gly Gln Gly Asn Gly Asn Gly Gly
145                 150                 155                 160

Arg Pro Ser Ser Ser Tyr Gly Ala Pro Gly Gly Gly Asn Gly Gly Arg
                165                 170                 175

Pro Ser Asp Thr Tyr Gly Ala Pro Gly Gly Gly Asn Gly Gly Arg Pro
            180                 185                 190

Ser Asp Thr Tyr Gly Ala Pro Gly Gly Gly Asn Asn Gly Gly Arg Pro
        195                 200                 205

Ser Ser Ser Tyr Gly Ala Pro Gly Gly Gly Asn Gly Gly Arg Pro Ser
    210                 215                 220

Asp Thr Tyr Gly Ala Pro Gly Gly Gly Asn Gly Asn Gly Ser Gly Gly
225                 230                 235                 240

Arg Pro Ser Ser Ser Tyr Gly Ala Pro Gly Gln Gly Gln Gly Gly Phe
                245                 250                 255

Gly Gly Arg Pro Ser Asp Ser Tyr Gly Ala Pro Gly Gln Asn Gln Lys
            260                 265                 270

Pro Ser Asp Ser Tyr Gly Ala Pro Gly Ser Gly Asn Gly Asn Gly Gly
        275                 280                 285

Arg Pro Ser Ser Ser Tyr Gly Ala Pro Gly Ser Gly Pro Gly Gly Arg
    290                 295                 300

Pro Ser Asp Ser Tyr Gly Pro Pro Ala Ser Gly
305                 310                 315

<210>  7
<211>  282
<212>  PRT
<213>  Artificial Sequence

<220>
<223>  elastin short

<400>  7

Met His His His His His His Ser Ser Gly Ser Ser Leu Gly Val Ser
1               5                   10                  15

Ala Gly Ala Val Val Pro Gln Pro Gly Ala Gly Val Lys Pro Gly Lys
            20                  25                  30

Val Pro Gly Val Gly Leu Pro Gly Val Tyr Pro Gly Gly Val Leu Pro
        35                  40                  45

Gly Ala Arg Phe Pro Gly Val Gly Val Leu Pro Gly Val Pro Thr Gly
    50                  55                  60

Ala Gly Val Lys Pro Lys Ala Pro Gly Val Gly Gly Ala Phe Ala Gly
65                  70                  75                  80

Ile Pro Gly Val Gly Pro Phe Gly Gly Pro Gln Pro Gly Val Pro Leu
                85                  90                  95

Gly Tyr Pro Ile Lys Ala Pro Lys Leu Pro Gly Gly Tyr Gly Leu Pro
            100                 105                 110

Tyr Thr Thr Gly Lys Leu Pro Tyr Gly Tyr Gly Pro Gly Gly Val Ala
        115                 120                 125

Gly Ala Ala Gly Lys Ala Gly Tyr Pro Thr Gly Thr Gly Val Gly Pro
    130                 135                 140

Gln Ala Ala Ala Ala Ala Ala Ala Lys Ala Ala Ala Lys Phe Gly Ala
145                 150                 155                 160

Gly Ala Ala Gly Val Leu Pro Gly Val Gly Gly Ala Gly Val Pro Gly
                165                 170                 175

Val Pro Gly Ala Ile Pro Gly Ile Gly Gly Ile Ala Gly Val Gly Thr
            180                 185                 190

Pro Ala Ala Ala Ala Ala Ala Ala Ala Ala Ala Lys Ala Ala Lys Tyr
        195                 200                 205

Gly Ala Ala Ala Gly Leu Val Pro Gly Gly Pro Gly Phe Gly Pro Gly
    210                 215                 220

Val Val Gly Val Pro Gly Ala Gly Val Pro Gly Val Gly Val Pro Gly
225                 230                 235                 240

Ala Gly Ile Pro Val Val Pro Gly Ala Gly Ile Pro Gly Ala Ala Val
                245                 250                 255

Pro Gly Val Val Ser Pro Glu Ala Ala Ala Lys Ala Ala Ala Lys Ala
            260                 265                 270

Ala Lys Tyr Gly Ala Arg Pro Gly Val Gly
        275                 280

<210>  8
<211>  12
<212>  PRT
<213>  Artificial Sequence

<220>
<223>  HisTag

<400>  8

Met His His His His His His Ser Ser Gly Ser Ser
1               5                   10

## Description

### Technical Field

The present invention relates to a method for purifying a recombinant protein from a recombinant cell expressing the recombinant protein.

### Background Art

Production of a protein of interest on an industrial scale is made possible by using genetically modified host cells. Many methods for isolating and purifying recombinant proteins produced by recombinant cells have been reported.

As methods for isolating an insoluble protein of interest, a method in which a metal hydroxide such as sodium hydroxide is used from a suspension of recombinant cells (Patent Literature 1), and a method in which an organic acid such as formic acid or propionic acid is used (Patent Literature 2) have been reported. However, when isolating an insoluble protein of interest by these methods, one problem was that the purity of the isolated protein of interest was not high, since it is difficult to remove impurities present with the protein of interest. Moreover, in the method using an organic acid, one problem was that isolatable proteins are limited, since a protein that is not resistant to acid is easily decomposed.

As a method that can remove impurities present with the protein of interest and does not use an organic acid, a method that dissolves in an aprotic polar solvent such as dimethylsulfoxide, removes impurities using a solvent such as water, and purifies the protein of interest (Patent Literature 3) has been reported. According to this method, the protein of interest can be purified to about 70%, but one problem was that the protein of interest is limited to hydrophilic recombinant proteins having a hydropathy index (HI) of 0 or less. In addition, gelation may also occur depending on the protein due to the influence of the aqueous solvent such as water to be added for removing impurities.

### Citation List

### Patent Literature

Patent Literature 1: Japanese Unexamined Patent Publication No. 2013-523665
Patent Literature 2: Japanese Unexamined Patent Publication No. 2004-503204
Patent Literature 3: International Publication No. WO 2014/103847

### Summary of Invention

### Technical Problem

In industrial scale production, a method for easily purifying recombinant proteins of interest with high purity is still desired. Therefore, the present invention aims to provide a method for easily purifying recombinant proteins with high purity from recombinant cells expressing intracellularly the recombinant proteins of interest as an insoluble matter.

### Solution to Problem

As a result of intensive studies, the present inventors have found that by treating a recombinant cell using an aprotic polar solvent under conditions where a protein derived from a host cell dissolves but the recombinant protein of interest does not dissolve, it is possible to easily purify a recombinant protein of interest with high purity without adding an aqueous solvent such as water, by removing the protein derived from the host cell before dissolving the recombinant protein of interest, and completed the present invention.

That is, the present invention relates, for example, to each of the following inventions.
[1] A method for purifying a recombinant protein of interest from a recombinant cell expressing intracellularly the recombinant protein as an insoluble matter,
   comprising treating the recombinant cell under conditions where a protein derived from a host cell dissolves in a first aprotic polar solvent to which an inorganic salt is added or not but the recombinant protein does not dissolve, removing a first soluble fraction, and then treating the resulting first insoluble fraction under conditions where the recombinant protein dissolves in a second aprotic polar solvent to which an inorganic salt is added.
[2] The method for purifying a recombinant protein according to [1], further comprising, after the treatment under conditions where the recombinant protein dissolves in the second aprotic polar solvent to which an inorganic salt is added, removing a second insoluble fraction to obtain a second soluble fraction containing the recombinant protein.
[3] The method for purifying a recombinant protein according to [2], further comprising treating the second soluble fraction obtained by removing the second insoluble fraction using a poor solvent for the recombinant protein, thereby aggregating the recombinant protein to obtain the purified recombinant protein as an aggregate.
[4] A method for purifying a recombinant protein, comprising the following steps (A) to (D):
   (A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
   (B) a step of removing a first soluble fraction and recovering a first insoluble fraction containing the recombinant protein;
   (C) a step of treating the recovered first insoluble fraction using a second aprotic polar solvent to which an inorganic salt is added, under conditions where the recombinant protein dissolves; and
   (D) a step of removing a second insoluble fraction to obtain a second soluble fraction containing the recombinant protein.
[5] The method for purifying a recombinant protein according to [4], further comprising:
   (E) a step of treating the second soluble fraction using a poor solvent for the recombinant protein, thereby aggregating the recombinant protein and recovering the purified recombinant protein as an aggregate.
[6] The method for purifying a recombinant protein according to any one of [1] to [5], wherein the conditions are a temperature condition.
[7] The method for purifying a recombinant protein according to any one of [1] to [6], wherein the first or second aprotic polar solvent has a dipole moment of 3.0 D or more.
[8] The method for purifying a recombinant protein according to any one of [1] to [7], wherein the inorganic salt is selected from the group consisting of alkali metal halides, alkaline earth metal halides, alkaline earth metal nitrates and thiocyanate.
[9] The method for purifying a recombinant protein according to any one of [1] to [8], wherein the recombinant protein is a structural protein.
[10] The method according to [9], wherein the structural protein is derived from a protein selected from the group consisting of collagen, elastin, resilin, silkworm silk and spider silk.
[11] A method for producing an artificial polypeptide fiber, comprising the following steps (A) to (D) and (F):
   (A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
   (B) a step of removing a first soluble fraction and recovering a first insoluble fraction containing the recombinant protein;
   (C) a step of treating the recovered first insoluble fraction using a second aprotic polar solvent to which an inorganic salt is added, under conditions where the recombinant protein dissolves;
   (D) a step of removing a second insoluble fraction to obtain a second soluble fraction containing the recombinant protein; and
   (F) a step of applying the second soluble fraction as a filamentous liquid to a coagulation liquid, thereby coagulating the recombinant protein in filament form, and recovering the recombinant protein to obtaining an undrawn yarn.
[12] A method for producing a polypeptide film, comprising the following steps (A) to (D) and (G):
   (A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
   (B) a step of removing a first soluble fraction and recovering a first insoluble fraction containing the recombinant protein;
   (C) a step of treating the recovered first insoluble fraction using a second aprotic polar solvent to which an inorganic salt is added, under conditions where the recombinant protein dissolves;
   (D) a step of removing a second insoluble fraction to obtain a second soluble fraction containing the recombinant protein; and
   (G) a step of forming a coating film of a predetermined thickness on a flat plate resistant to the second aprotic polar solvent using the second soluble fraction, and removing the second aprotic polar solvent from the coating film to obtain a polypeptide film.

### Advantageous Effects of Invention

Although the method for purifying a recombinant protein of the present invention is a simple method with no complicated steps and few steps, the purified recombinant protein obtained is high in purity and can be used as it is for production such as spinning and film formation, without further undergoing a purification step.

In addition, the method for purifying a recombinant protein of the present invention does not require a step of adding an acid. Therefore, the recombinant protein to be purified is not limited to proteins resistant to acid.

Furthermore, the method for purifying a recombinant protein of the present invention does not require a purification step which replaces the aprotic polar solvent containing the recombinant protein with an aqueous solvent such as water. Therefore, the recombinant protein to be purified is not limited to hydrophilic recombinant proteins having a hydropathy index of 0 or less. Also, there is no worry of gelation of the recombinant protein due to the influence of the aqueous solvent. In addition, since no aqueous solvent such as water is added to the aprotic polar solvent, it is also possible to recover the aprotic polar solvent and reuse it.

### Brief Description of Drawings

Figure 1 is a photograph showing the results of the analysis by polyacrylamide gel electrophoresis (SDS-PAGE) regarding the removal of the protein derived from a host cell examined in Example 1.
Figure 2 is a photograph showing the results of the analysis by SDS-PAGE of the supernatant fraction obtained by centrifugation, regarding the removal of the protein derived from a host cell examined in Example 2.
Figure 3 is a photograph showing the results of the analysis by SDS-PAGE of the precipitate fraction obtained by centrifugation, regarding the removal of the protein derived from a host cell examined in Example 2.
Figure 4 is a photograph showing the results of the analysis by SDS-PAGE of the supernatant fraction obtained by centrifugation, regarding the removal of the protein derived from a host cell examined in Example 3.
Figure 5 is a photograph showing the results of the analysis by SDS-PAGE of the precipitate fraction obtained by centrifugation regarding the removal of the protein derived from a host cell examined in Example 3.
Figure 6 is a photograph showing the results of the analysis by SDS-PAGE regarding the purification of the protein of interest examined in Example 4.
Figure 7 is a photograph showing the results of the analysis by SDS-PAGE regarding the purification of the protein of interest examined in Example 5.
Figure 8 is a photograph showing the results of the analysis by SDS-PAGE regarding the purification of the protein of interest examined in Example 6.
Figure 9 is a photograph showing the results of the analysis by SDS-PAGE regarding the purification of the protein of interest examined in Example 7.
Figure 10 is a photograph of a yarn formed using the purified protein solution as a dope for spinning, which was examined in Example 8.
Figure 11 is a photograph showing the state of gelation of the protein of interest examined in Example 9.
Figure 12 is a photograph showing the results of the analysis by SDS-PAGE regarding the purification of the protein of interest by a conventional method and the method of Example 9, examined in the present Example.

### Description of Embodiments

The following describes in detail the embodiments of the present invention. However, the present invention is not limited to the following embodiments.

The method for purifying a recombinant protein according to one embodiment is a method for purifying a recombinant protein of interest from a recombinant cell expressing intracellularly the recombinant protein as an insoluble matter, comprising treating the recombinant cell under conditions where a protein derived from a host cell dissolves in a first aprotic polar solvent to which an inorganic salt is added or not but the recombinant protein does not dissolve, removing a first soluble fraction, and then treating the resulting first insoluble fraction under conditions where the recombinant protein dissolves in a second aprotic polar solvent to which an inorganic salt is added. It is preferable that the method further comprise, after the treatment under conditions where the recombinant protein dissolves in a second aprotic polar solvent to which an inorganic salt is added, removing a second insoluble fraction to obtain a second soluble fraction containing the recombinant protein. It is preferable that the method further comprise treating the second soluble fraction obtained by removing the second insoluble fraction using a poor solvent for the recombinant protein, thereby aggregating the recombinant protein to obtain the purified recombinant protein as an aggregate.

Specifically, the method for purifying a recombinant protein according to one embodiment comprises the following steps (A) to (D):
(A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
(B) a step of removing a first soluble fraction and recovering a first insoluble fraction containing the recombinant protein;
(C) a step of treating the recovered first insoluble fraction using a second aprotic polar solvent to which an inorganic salt is added, under conditions where the recombinant protein dissolves; and
(D) a step of removing a second insoluble fraction to obtain a second soluble fraction containing the recombinant protein.

### (Recombinant protein)

The recombinant protein of interest according to the present embodiment (hereinafter also referred to as "recombinant protein" or "protein of interest ") can be any protein preferable for the production on an industrial scale, and examples thereof include proteins that can be used for industrial purposes, proteins that can be used for medical purposes and structural proteins. Specific examples of proteins that can be used for industrial or medical purposes include enzymes, regulatory proteins, receptors, peptide hormones, cytokines, membranes or transport proteins, antigens used for vaccination, vaccines, antigen binding proteins, immunostimulatory proteins, allergens, full-length antibodies or antibody fragments, or derivatives. Specific examples of structural proteins include spider silk, silkworm silk, collagen, elastin and resilin, as well as proteins derived therefrom.

Examples of proteins derived from spider silk or silkworm silk which are a fibroin proteins include proteins containing a domain sequence represented by Formula 1: [(A)ₙ motif-REP]ₘ, wherein (A)ₙ motif represents an amino acid sequence composed of 4 to 20 amino acid residues and the proportion of the number of alanine residues to the total number of amino acid residues in (A)ₙ motif is 80% or more; REP represents an amino acid sequence composed of 10 to 200 amino acid residues; and m represents an integer of 8 to 300; and a plurality of (A)ₙ motifs may be the same amino acid sequence or different amino acid sequences, and a plurality of REP may be the same amino acid sequence or different amino acid sequences. Specifically, these include proteins containing the amino acid sequence represented by SEQ ID NO:1 (PRT 468) to SEQ ID NO:4. The hydropathy index of PRT 468 (SEQ ID NO:1) is -0.59 and the hydropathic index of PRT 698 (SEQ ID NO:4) is 0.43. The value of the hydropathy index is the value calculated according to the method described in International Publication No. WO 2014/103846.

Examples of proteins derived from collagen include proteins containing a domain sequence represented by Formula 2: [REP 2]ₒ, wherein o represents an integer of 5 to 300; and REP 2 represents an amino acid sequence composed of Gly-X-Y and X and Y represent any amino acid residue other than Gly; and a plurality of REP2 may be the same amino acid sequence or different amino acid sequences. Specifically, these include proteins containing the amino acid sequence represented by SEQ ID NO:5 (Collagen-type 4-Kai). The amino acid sequence represented by SEQ ID NO:5 is an amino acid sequence with the amino acid sequence represented by SEQ ID NO:8 (His tag sequence and hinge sequence) added to the N-terminal of the amino acid sequence at residue positions 301 to 540 corresponding to the repeat portion and the motif of a partial sequence of human collagen type 4 obtained from the NCBI database (NCBI Genbank Accession Number: CAA56335.1, GI: 3702452). The hydropathy index of Collagen-type 4-Kai is -0.75.

Examples of proteins derived from resilin include proteins containing a domain sequence represented by Formula 3: [REP 3]ₚ, wherein p represents an integer of 4 to 300; REP 3 represents an amino acid sequence composed of Ser-J-J-Tyr-Gly-U-Pro, where J represents any amino acid residue and in particular, it is preferable that it be an amino acid residue selected from the group consisting of Asp, Ser and Thr; and U is any amino acid residue and in particular, it is preferable that it be an amino acid residue selected from the group consisting of Pro, Ala, Thr and Ser; and a plurality of REP3 may be the same amino acid sequence or different amino acid sequences. Specifically, these include proteins containing the amino acid sequence represented by SEQ ID NO:6. The amino acid sequence represented by SEQ ID NO:6 is an amino acid sequence in which Thr at residue position 87 is replaced with Ser in the amino acid sequence of resilin (NCBI Genbank Accession No. NP 611157.1, GI: 24654243), and an amino acid sequence represented by SEQ ID NO:8 (His tag sequence and hinge sequence) is added to the N-terminal of the amino acid sequence at residue positions 19 to 321 of the sequence in which Asn at residue position 95 is replaced with Asp. The hydropathy index of Resilin-Kai (SEQ ID NO:6) is -1.22.

Examples of proteins derived from elastin include proteins having amino acid sequences such as NCBI Genbank accession numbers AAC98395 (human), I47076 (sheep) and NP786966 (bovine). Specifically, these include proteins containing the amino acid sequence represented by SEQ ID NO:7. The amino acid sequence represented by SEQ ID NO:7 is an amino acid sequence with the amino acid sequence represented by SEQ ID NO:8 (His tag sequence and hinge sequence) added to the N-terminal of the amino acid sequence at residue positions 121 to 390 in the amino acid sequence of NCBI Genbank Accession No. AAC98395. The hydropathy index of elastin Homo sapiens (SEQ ID NO:7) is 0.42.

### (Recombinant cell)

The recombinant cell according to this embodiment is a recombinant cell expressing intracellularly a recombinant protein as an insoluble matter and can be obtained by a general method using a genetic engineering technique.

The recombinant cell can be obtained, for example, by transforming a host (host cell) with an expression vector having a nucleic acid sequence encoding a protein of interest and one or more regulatory sequences operably linked to the nucleic acid sequence.

The regulatory sequence is a sequence that controls the expression of a recombinant protein in a host (for example, promoters, enhancers, ribosome binding sequences, transcription termination sequences, etc.), and can be appropriately selected according to the type of host. The type of expression vector can be appropriately selected according to the type of host, such as plasmid vectors, virus vectors, cosmid vectors, fosmid vectors and artificial chromosome vectors.

Both prokaryotes and eukaryotes such as yeasts, filamentous fungi, insect cells, animal cells and plant cells can be suitably used as a host. Preferable examples of prokaryotes include bacteria such as Escherichia coli, Bacillus subtilis, Pseudomonas, Corynebacterium, Lactococcus, and more preferably Escherichia coli cells.

As the expression vector, a vector able to autonomously replicate in a host cell or able to be incorporated into a chromosome of a host, and having a promoter at a position capable of transcribing a nucleic acid encoding a protein of interest is suitably used.

When a prokaryote such as a bacterium is used as a host, it is preferable that the expression vector according to the present invention be a vector capable of autonomous replication in prokaryotic cells, and at the same time, containing a promoter, a ribosome binding sequence, a nucleic acid sequence according to the present invention and a transcription termination sequence. A gene sequence controlling the promoter may be contained.

As the promoter, an inducible promoter which functions in a host cell and is capable of inducing the expression of a protein of interest may be used. An inducible promoter is a promoter capable of controlling transcription by physical factors such as the presence of an inducer (expression inducer), the absence of a repressor molecule, or the increase or decrease in temperature, osmotic pressure or pH value.

Examples of prokaryotic hosts such as bacteria include microorganisms belonging to the genus Escherichia, Brevibacillus, Serratia, Bacillus, Microbacterium, Brevibacterium, Corynebacterium and Pseudomonas.

Examples of microorganisms belonging to the genus Escherichia include Escherichia coli BL21 (Novagen), Escherichia coli BL21 (DE3) (Life Technologies), Escherichia coli BLR (DE3) (Merck Millipore), Escherichia coli DH1, Escherichia coli GI698, Escherichia coli HB101, Escherichia coli JM109, Escherichia coli K5 (ATCC 23506), Escherichia coli KY3276, Escherichia coli MC1000, Escherichia coli MG1655 (ATCC 47076), Escherichia coli No.49, Escherichia coli Rosetta (DE3) (Novagen), Escherichia coli TB1, Escherichia coli Tuner (Novagen), Escherichia coli Tuner (DE3) (Novagen), Escherichia coli W1485, Escherichia coli W3110 (ATCC 27325), Escherichia coli XL1-Blue and Escherichia coli XL2-Blue.

Examples of microorganisms belonging to the genus Brevibacillus include Brevibacillus agri, Brevibacillus borstelensis, Brevibacillus centrosporus, Brevibacillus formosus, Brevibacillus invocatus, Brevibacillus laterosporus, Brevibacillus limnophilus, Brevibacillus parabrevis, Brevibacillus reuszeri, Brevibacillus thermoruber, Brevibacillus brevis 47 (FERM BP-1223), Brevibacillus brevis 47K (FERM BP-2308), Brevibacillus brevis 47-5 (FERM BP-1664), Brevibacillus brevis 47-5Q (JCM8975), Brevibacillus choshinensis HPD31 (FERM BP-1087), Brevibacillus choshinensis HPD31-S (FERM BP-6623), Brevibacillus choshinensis HPD31-OK (FERM BP-4573) and Brevibacillus choshinensis SP3 strain (Takara Co., Ltd.).

Examples of microorganisms belonging to the genus Serratia include Serratia liquefacience ATCC 14460, Serratia entomophila, Serratia ficaria, Serratia fonticola, Serratia grimesii, Serratia proteamaculans, Serratia odorifera, Serratia plymuthica and Serratia rubidaea.

Examples of microorganisms belonging to the genus Bacillus include Bacillus subtilis and Bacillus amyloliquefaciens.

Examples of microorganisms belonging to the genus Microbacterium include Microbacterium ammoniaphilum ATCC15354.

Examples of microorganisms belonging to the genus Brevibacterium include Brevibacterium divaricatum (Corynebacterium glutamicum) ATCC 14020, Brevibacterium flavum (Corynebacterium glutamicum ATCC 14067) ATCC 13826, ATCC 14067, Brevibacterium immariophilum ATCC 14068, Brevibacterium lactofermentum (Corynebacterium glutamicum ATCC 13869) ATCC 13665, ATCC 13869, Brevibacterium roseum ATCC 13825, Brevibacterium saccharolyticum ATCC 14066, Brevibacterium thiogenitalis ATCC 19240, Brevibacterium album ATCC 15111 and Brevibacterium cerinumATCC 15112.

Examples of microorganisms belonging to the genus Corynebacterium include Corynebacterium ammoniagenes ATCC 6871, ATCC 6872, Corynebacterium glutamicum ATCC 13032, Corynebacterium glutamicum ATCC 14067, Corynebacterium acetoacidophilum ATCC 13870, Corynebacterium acetoglutamicum ATCC 15806, Corynebacterium alkanolyticum ATCC 21511, Corynebacterium carnae ATCC 15991, Corynebacterium glutamicum ATCC 13020, ATCC 13032, ATCC 13060, Corynebacterium lilium ATCC15990, Corynebacterium melassecola ATCC 17965, Corynebacterium thermoaminogenes AJ12340 (FERMBP-1539) and Corynebacterium herculis ATCC13868.

Examples of microorganisms belonging to the genus Pseudomonas include Pseudomonas putida, Pseudomonas fluorescens, Pseudomonas brassicacearum, Pseudomonas fulva, and Pseudomonas sp. D-0110.

As a method for introducing the expression vector into the host cell, any method can be used as long as it introduces DNA into the host cell. Examples include the method using calcium ions [Proc. Natl. Acad. Sci. USA, 69, 2110 (1972)], the protoplast method (Japanese Unexamined Patent Publication No. S63-248394), or the method described in Gene, 17, 107 (1982) and Molecular & General Genetics, 168, 111 (1979).

The transformation of a microorganism belonging to the genus Brevibacillus can be carried out, for example, by the method of Takahashi et al. (J. Bacteriol., 1983, 156: 1130-1134), the method of Takagi et al. (Agric. Biol. Chem., 1989, 53: 3099 -3100) or the method of Okamoto et al. (Biosci. Biotechnol. Biochem., 1997, 61: 202-203).

Examples of vectors for introducing a nucleic acid encoding a protein of interest (hereinafter simply referred to as "vectors") include pBTrp2, pBTac1, pBTac2 (all commercially available from Boehringer Mannheim), pKK 233-2 (manufactured by Pharmacia), pSE280 (manufactured by Invitrogen), pGEMEX-1 (manufactured by Promega), pQE-8 (manufactured by QIAGEN), pKYP10 (Japanese Unexamined Patent Publication No. S58-110600), pKYP200 [Agric. Biol. Chem., 48, 669 (1984)], pLSA 1 [Agric. Biol. Chem., 53, 277 (1989)], pGEL1 [Proc. Natl. Acad. Sci. USA, 82, 4306 (1985), pBluescript II SK (-) (manufactured by Stratagene), pTrs30 [prepared from Escherichia coli JM109/pTrS30 (FERM BP-5407)], pTrs32 [prepared from Escherichia coli JM109/pTrS32 (FERM BP-5408], pGHA2 [prepared from Escherichia coli IGHA2 (FERM B-400), Japanese Unexamined Patent Publication No. S60-221091], pGKA2 [prepared from Escherichia coli IGKA 2 (FERM BP-6798), Japanese Unexamined Patent Publication No. S60-221091], pTerm2 (US 4686191, US 4939094, US 5160735), pSupex, pUB110, pTP5, pC194, pEG400 [J. Bacteriol., 172, 2392 (1990)], pGEX (manufactured by Pharmacia) and pET system (manufactured by Novagen).

When using Escherichia coli as a host, examples of suitable vectors include pUC18, pBluescriptII, pSupex, pET22b and pCold.

Specific examples of vectors suitable for microorganisms belonging to the genus Brevibacillus include pUB110, which is known as a Bacillus subtilis vector, or pHY500 (Japanese Unexamined Patent Publication No. H2-31682), pNY700 (Japanese Unexamined Patent Publication No. H4-278091), pHY4831 (J. Bacteriol., 1987, 1239-1245), pNU200 (Shigezou Udaga, Japan Society for Bioscience, Biotechnology and Agrochemistry Journal 1987, 61: 669-676), pNU100 (Appl. Microbiol. Biotechnol., 1989, 30: 75-80), pNU211 (J. Biochem., 1992, 112: 488-491), pNU211R2L5 (Japanese Unexamined Patent Publication No. H7-170984), pNH301 (Appl. Environ. Microbiol., 1992, 58: 525-531), pNH326, pNH400 (J. Bacteriol., 1995, 177: 745-749), pHT210 (Japanese Unexamined Patent Publication No. H6-133782), pHT110R2L5 (Appl. Microbiol. Biotechnol., 1994, 42: 358-363), or pNCO2 (Japanese Unexamined Patent Publication No. 2002-238569), which is a shuttle vector of microorganisms belonging to the genus Escherichia coli and Brevibacillus.

The promoter when a prokaryote is used as a host is not limited as long as it functions in a host cell. Examples include promoters derived from Escherichia coli or phages such as trp promoters (Ptrp), lac promoters, PL promoters, PR promoters and T7 promoters. Moreover, promoters designed and modified artificially such as promoters in which two Ptrp are connected in series (Ptrp × 2), tac promoters, lac T7 promoters and let I promoters can also be used. It is preferable to use a plasmid in which the distance between the Shine-Dalgarno sequence, which is a ribosome binding sequence, and the initiation codon is adjusted to an appropriate distance (for example, 6 to 18 bases). In the expression vector, a transcription termination sequence is not necessarily required for the expression of the nucleic acid, but it is preferable to place a transcription termination sequence immediately below the structural gene.

Examples of eukaryotic hosts include yeasts, filamentous fungi (mold etc.) and insect cells.

Examples of yeasts include yeasts belonging to the genera Saccharomyces, Schizosaccharomyces, Kluyveromyces, Trichosporon, Schwanniomyces, Pichia, Candida, Yarrowia and Hansenula. More specifically, these include Saccharomyces cerevisiae, Schizosaccharomyces pombe, Kluyveromyces lactis, Kluyveromyces marxianus, Trichosporon pullulans, Schwanniomyces alluvius, Schwanniomyces occidentalis, Candida utilis, Pichia pastoris, Pichia angusta, Pichia methanolica, Pichia polymorpha, Pichia stipitis, Yarrowia lipolytica and Hansenula polymorpha.

It is usually preferable that the expression vector when yeast is used as a host cell include an origin of replication (if amplification in the host is required), a selectable marker for propagating the vector in Escherichia coli, a promoter and a terminator for recombinant protein expression in the yeast, and a selectable marker for yeast.

When the expression vector is a nonintegrated vector, it is preferable to further include an autonomously replicating sequence (ARS). This allows improving the stability of the expression vector in the cell (Myers, A. M., et al. (1986) Gene 45: 299-310).

Examples of vectors when using yeast as a host include YEP13 (ATCC 37115), YEp24 (ATCC 37051), YCp50 (ATCC 37419), YIp, pHS19, pHS15, pA0804, pHIL3Ol, pHIL-S1, pPIC9K, pPICZα, pGAPZα and pPICZ B.

Specific examples of the promoter when yeast is used as a host are not limited as long as they can be expressed in yeast. Examples include promoters of glycolytic genes such as hexose kinase, PHO5 promoter, PGK promoter, GAP promoter, ADH promoter, gal 1 promoter, gal 10 promoter, heat shock polypeptide promoter, MFα 1 promoter, CUP 1 promoter, pGAP promoter, pGCW14 promoter, AOX1 promoter and MOX promoter.

As a method for introducing an expression vector into yeast, any method can be used as long as it introduces DNA into yeast, and examples include the electroporation method (Methods Enzymol., 194, 182 (1990)), the spheroplast method (Proc. Natl. Acad. Sci., USA, 81, 4889 (1984)), and the lithium acetate method (J. Bacteriol., 153, 163 (1983)), Proc. Natl. Acad. Sci. USA, 75, 1929 (1978).

Examples of filamentous fungi include the fungi belonging to the genera Acremonium, Aspergillus, Ustilago, Trichoderma, Neurospora, Fusarium, Humicola, Penicillium, Myceliophtora, Botryts, Magnaporthe, Mucor, Metarhizium, Monascus, Rhizopus and Rhizomucor.

Specific examples of filamentous fungi include Acremonium alabamense, Acremonium cellulolyticus, Aspergillus aculeatus, Aspergillus awamori, Aspergillus oryzae, Aspergillus sake, Aspergillus sojae, Aspergillus tubigensis, Aspergillus niger, Aspergillus nidulans, Aspergillus parasiticus, Aspergillus ficuum, Aspergillus phoeicus, Aspergillus foetidus, Aspergillus flavus, Aspergillus fumigatus, Aspergillus japonicus, Trichoderma viride, Trichoderma harzianum, Trichoderma reseei, Chrysosporium lucknowense, Thermoascus, Sporotrichum, Sporotrichum cellulophilum, Talaromyces, Thielavia terrestris, Thielavia, Neurospora crassa, Fusarium oxysporus, Fusarium graminearum, Fusarium venenatum, Humicola insolens, Penicillium chrysogenum, Penicillium camemberti, Penicillium canescens, Penicillium emersonii, Penicillium funiculosum, Penicillium griseoroseum, Penicillium purpurogenum, Penicillium roqueforti, Myceliophtaora thermophilum, Mucor ambiguus, Mucor circinelloides, Mucor fragilis, Mucor hiemalis, Mucor inaequisporus, Mucor oblongiellipticus, Mucor racemosus, Mucor recurvus, Mocor saturninus, Mocor subtilissmus, Ogataea polymorpha, Phanerochaete chrysosporium, Rhizomucor miehei, Rhizomucor pusillus and Rhizopus arrhizus.

Specific examples of promoters when filamentous fungi are used as hosts may be either genes related to the glycolytic pathway, genes related to constitutive expression, or enzymatic genes related to hydrolysis, and specific examples thereof include amyB, glaA, agdA, glaB, TEF 1, xynF1tannasegene, No.8AN, gpdA, pgkA, enoA, melO, sodM, catA and catB.

The introduction of an expression vector into filamentous fungi can be carried out using a conventionally known method. Examples include the method of Cohen et al. (Calcium chloride method) [Proc. Natl. Acad. Sci. USA, 69: 2110 (1972)], the protoplast method [Mol. Gen. Genet., 168: 111 (1979)], the competent method [J. Mol. Biol., 56: 209 (1971)] and the electroporation method.

Examples of insect cells include lepidopteran insect cells, more specifically insect cells derived from Spodoptera frugiperda such as Sf9 and Sf21, and insect cells derived from Trichoplusia ni such as High 5.

Examples of vectors when insect cells are used as a host include baculoviruses such as the Autographa californica nuclear polyhedrosis virus which is a virus that infects cabbage moths (Baculovirus Expression Vectors, A Laboratory Manual, W. H. Freeman and Company, New York (1992)).

When insect cells are used as a host, polypeptides can be expressed by the methods described, for example, in Current Protocols in Molecular Biology, Baculovirus Expression Vectors, A Laboratory Manual, W H. Freeman and Company, New York (1992) and Bio/Technology, 6, 47 (1988). That is, after a recombinant gene transfer vector and a baculovirus are transferred into an insect cell to obtain a recombinant virus (expression vector) in the insect cell culture supernatant, the insect cell can be further infected with the recombinant virus and the polypeptide can be expressed. Examples of gene transfer vectors used in this method include pVL1392, pVL1393 and pBlueBacIII (all manufactured by Invitrogen).

Examples of methods for transferring a recombinant gene transfer vector and a baculovirus into an insect cell for preparing a recombinant virus include the calcium phosphate method (Japanese Unexamined Patent Publication No. H2-227075) and the lipofection method (Proc. Natl. Acad. Sci. USA, 84, 7413 (1987)).

It is preferable that the recombinant vector further contain a selectable marker gene for selecting a transformant. For example, in Escherichia coli, resistance genes for various drugs such as tetracycline, ampicillin and kanamycin can be used as selectable marker genes. Recessive selectable markers capable of complementing genetic mutations involved in auxotrophy can also be used. In yeasts, as a selectable marker gene, a resistance gene against geneticin can be used, and genes complementing a genetic mutation involved in auxotrophy, selectable markers such as LEU2, URA3, TRP1 and HIS3 can also be used. In filamentous fungi, examples of selectable marker genes include marker genes selected from the group consisting of niaD (Biosci. Biotechnol. Biochem., 59, 1795-1797 (1995)), argB (Enzyme Microbiol Technol, 6, 386-389, (1984)), sC (Gene, 84, 329-334, (1989)), ptrA (Biosci Biotechnol Biochem, 64, 1416-1421, (2000)), pyrG (Biochem Biophys Res Commun, 112, 284-289, (1983)), amdS (Gene, 26, 205-221, (1983)), aureobasidin resistance gene (Mol Gen Genet, 261, 290-296, (1999)), benomyl resistance gene (Proc Natl Acad Sci USA, 83, 4869-4873, (1986)) and hygromycin resistance gene (Gene, 57, 21-26, (1987)), and leucine requirement complementing genes. Moreover, when the host is an auxotrophic mutant strain, a wild-type gene complementing the auxotrophy can also be used as the selectable marker gene.

The host transformed with the expression vector can be selected by plaque hybridization using a probe selectively binding to the nucleic acid, colony hybridization, or the like. As the probe, one in which a partial DNA fragment amplified by PCR method has been modified with a radioisotope or digoxigenin based on sequence information of the nucleic acid, can be used.

### (Production of recombinant protein)

Recombinant proteins can be produced by culturing a host transformed by the expression vector in a culture medium. The method for culturing the host in the culture medium can be carried out according to the method usually used for culturing the host.

When the host is a prokaryote such as Escherichia coli or a eukaryote such as yeast, as a culture medium, either a natural medium or a synthetic medium may be used, as long as it is a medium that contains a carbon source, a nitrogen source, inorganic salts and the like that can be assimilated by the host, and that can efficiently carry out the culture of the host.

As the carbon source, any carbon source that can be assimilated by the host may be used, for example, carbohydrates such as glucose, fructose, sucrose, and molasses, starch and starch hydrolyzates containing these, organic acids such as acetic acid and propionic acid, and alcohols such as ethanol and propanol can be used.

As the nitrogen source, for example, ammonium, ammonium chloride, ammonium salts of inorganic acids or organic acids such as ammonium sulfate, ammonium acetate and ammonium phosphate, other nitrogen-containing compounds, and peptone, meat extract, yeast extract, corn steep liquor, casein hydrolyzate, soybean meal and soybean meal hydrolyzate, various fermented microbial cells and digested products thereof can be used.

As the inorganic salt, for example, monopotassium phosphate, dipotassium phosphate, magnesium phosphate, magnesium sulfate, sodium chloride, ferrous sulfate, manganese sulfate, copper sulfate and calcium carbonate can be used.

Prokaryotes such as Escherichia coli or eukaryotes such as yeast can be cultured, for example, under aerobic conditions such as shaking culture or deep aeration stirring culture. The culture temperature is, for example, 15 to 40°C. The culture time is usually 16 hours to 7 days. It is preferable to maintain the pH of the culture medium during the culture at 3.0 to 9.0. The pH of the culture medium can be adjusted by using an inorganic acid, an organic acid, an alkaline solution, urea, calcium carbonate, ammonia, or the like.

In addition, antibiotics such as ampicillin and tetracycline may be added to the culture medium as necessary during the culture. When culturing a microorganism transformed with an expression vector using an inducible promoter as the promoter, an inducer may be added to the medium as necessary. For example, when culturing a microorganism transformed with an expression vector using a lac promoter, isopropyl-P-D-thiogalactopyranoside or the like may be added to the medium, and when culturing a microorganism transformed with an expression vector using a trp promoter, indoleacrylic acid or the like may be added to the medium.

As a culture medium for insect cells, the commonly used TNM-FH medium (manufactured by Pharmingen), Sf-900 II SFM medium (manufactured by Life Technologies), ExCell 400, ExCell 405 (both manufactured by JRH Biosciences), Grace's Insect Medium (Nature, 195, 788 (1962)) and the like can be used.

The cultivation of insect cells can be carried out, for example, under conditions such as a culture medium pH of 6 to 7 and a culture temperature of 25 to 30°C, for a culture time of 1 to 5 days. In addition, antibiotics such as gentamicin may be added to the culture medium as necessary during the culture.

When the host is a plant cell, the transformed plant cell may be cultured as it is, or it can be cultured by differentiating it into a plant organ. As the culture medium for culturing the plant cells, commonly used Murashige-and-Scoog (MS) medium, White medium, or these media supplemented with plant hormones such as auxin and cytokinin can be used.

The cultivation of animal cells can be carried out, for example, under conditions such as a culture medium pH of 5 to 9 and a culture temperature of 20 to 40°C, for a culture time of 3 to 60 days. In addition, antibiotics such as kanamycin and hygromycin may be added to the medium as necessary during the culture.

According to the above method, a protein of interest can be expressed as an insoluble matter in a host cell.

### [Step (A)]

The step (A) is a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve.

A part of the protein derived from the host cell contained in the recombinant cell expressing intracellularly the recombinant protein of interest as an insoluble matter can be effectively dissolved in an aprotic polar solvent to which no inorganic salt is added or in an aprotic polar solvent to which an inorganic salt is added. The first aprotic polar solvent may be added to dried recombinant cells such as dried bacterial cells or may be added to a suspension or the like of recombinant cells. Furthermore, by treating under conditions where a protein derived from a host cell dissolves but the recombinant protein does not dissolve, the protein derived from the host cell dissolves and is contained in the soluble fraction (first soluble fraction), and the recombinant protein is contained in the insoluble fraction (first insoluble fraction). By removing the first soluble fraction in the following step (B), the recombinant protein can be separated from the protein derived from the host cell.

The recombinant cell when removing the protein derived from the host cell may be intact cells or cells after treatment such as disruption treatment. Moreover, the method of the present invention can also be applied to remove residual host cell derived proteins using cells that have already undergone a simple purification treatment.

### (First aprotic polar solvent)

Examples of the first aprotic polar solvent include dimethylsulfoxide (DMSO), N-methyl-2-pyrrolidone (NMP), 1,3-dimethyl-2-imidazolidone (DMI), N,N-dimethylformamide (DMF), N,N-dimethylacetamide (DMA), acetonitrile, acetone, isopropanol, propylene carbonate, hexamethylphosphoramide, N-ethylpyrrolidone, nitrobenzene, furfural, γ-butyrolactone, ethylene sulfite, sulfolane, succinonitrile, ethylene carbonate, but it is not limited thereto. In these aprotic polar solvents, those having a dipole moment of 3.0 D or more are preferable, for example, DMSO, NMP, DMI, DMF, DMA and acetonitrile are preferable, and DMSO, NMP, DMF and DMA are more preferable.

The purity of the aprotic polar solvent is not particularly limited, and is not required to be of high purity in this step, but it is preferable that the purity be 80% or more.

As the first aprotic polar solvent, one aprotic polar solvent may be used alone, or two or more aprotic polar solvents may be suitably mixed and used.

When dissolving polar compounds such as proteins, solvents with high polarity are effective, and molecules with a dipole moment of 3.0 D or more are said to have strong polarity. The dipole moments of organic compounds (organic solvents) based on the solvent handbook (Kodansha Scientific Co., 2007) are listed in Table 1.

**[Table 1]**

| Organic solvent | Dipole moment (D) | Organic solvent | Dipole moment (D) |
|---|---|---|---|
| DMSO | 4.30 | NMP (cyclic) | 4.09 |
| DMI | 4.05 | DMF | 3.86 |
| DMA | 3.72 | Acetonitrile | 3.44 |
| Acetone | 2.69 | Isopropanol | 1.68 |

The addition volume of the first aprotic polar solvent to be added to the recombinant cells may be any amount as long as it can dissolve the host cells, for example, per the recombinant cell dry weight (wt), the aprotic polar solvent (vol)/recombinant cell dry weight (wt) ratio may be 10 to 100 times, 12 to 50 times, or 20 to 35 times. Here, the recombinant cell dry weight means, for example, the weight of the dried bacteria cells when the host is a bacterium.

### (Inorganic salt)

Adding an inorganic salt to the first aprotic polar solvent makes the protein derived from the host cell more soluble.

Examples of inorganic salts to be added to the first aprotic polar solvent include alkali metal halides, alkaline earth metal halides, alkaline earth metal nitrates, thiocyanates and perchlorates.

Examples of alkali metal halides include potassium bromide, sodium bromide, lithium bromide, potassium chloride, sodium chloride, lithium chloride, sodium fluoride, potassium fluoride, cesium fluoride, potassium iodide, sodium iodide and lithium iodide.

Examples of alkaline earth metal halides include calcium chloride, magnesium chloride, magnesium bromide, calcium bromide, magnesium iodide and calcium iodide.

Examples of alkaline earth metal nitrates include calcium nitrate, magnesium nitrate, strontium nitrate and barium nitrate.

Examples of thiocyanates include sodium thiocyanate, ammonium thiocyanate and (guanidinium thiocyanate).

Examples of perchlorates include ammonium perchlorate, potassium perchlorate, calcium perchlorate, silver perchlorate, sodium perchlorate and magnesium perchlorate.

These inorganic salts may be used alone, or two or more of them may be used in combination.

Suitable inorganic salts include alkali metal halides and alkaline earth metal halides, and specific examples of suitable inorganic salts include lithium chloride and calcium chloride.

As the amount of an inorganic salt to be added, the optimum amount may be determined depending on the first aprotic polar solvent to be used, but for example, an inorganic salt of 0 to 1.0 M to the first aprotic polar solvent can be added. For example, the upper limit of the amount of the inorganic salt to be added may be 0.7 M, 0.6 M or 0.5 M, and the lower limit of the amount of the inorganic salt to be added may be 0.05 M, 0.1 M or 0.2 M.

When DMSO is used as the first aprotic polar solvent, the amount of the inorganic salt to be added is preferably 0 to 0.7 M, more preferably 0 to 0.3 M, and further preferably 0 to 0.1 M.

When DMF, DMA, DMI and NMP are used as the first aprotic polar solvent, the amount of the inorganic salt to be added is preferably 0 to 0.7 M, more preferably 0 to 0.6 M and further preferably 0 to 0.5 M.

The first aprotic polar solvent can further contain a solvent that inhibits the dissolution of the protein of interest. Adding an inorganic salt to the first aprotic polar solvent makes the protein derived from the host cell more soluble, but at the same time, tends to make the protein of interest also more soluble. Therefore, adding a solvent that inhibits the dissolution of the protein of interest to the first aprotic polar solvent allows to efficiently dissolve the protein derived from the host cell while preventing the dissolution of the protein of interest. As a solvent that inhibits the dissolution of the protein of interest, an aprotic polar solvent having a cyclic structure is preferable, and specific examples thereof include cyclic ketones, nitrogen-containing heterocyclic compounds, oxygen-containing heterocyclic compounds, sulfur-containing heterocyclic compounds and heterocyclic compounds having two or more atoms among nitrogen, oxygen and sulfur.

Examples of cyclic ketone solvents include cyclohexanone, cyclohexenone, isophorone, cyclopentanone and cyclopentenone, cyclohexanone and isophorone are preferable, and cyclohexanone is more preferable.

Examples of nitrogen-containing heterocyclic compound solvents include pyridine, piperidine, pyrrolidine, picoline, quinoline, NMP, 2-pyrrolidone, N-ethyl-2-pyrrolidone and DMI, pyridine, NMP, 2-pyrrolidone and DMI are preferable, and NMP is more preferable.

Examples of oxygen-containing heterocyclic compound solvents include dioxane, tetrahydrofuran, furfural, dioxolane, ethylene carbonate, propylene carbonate, δ-valerolactone and γ-butyrolactone, tetrahydrofuran, ethylene carbonate, propylene carbonate, δ-valerolactone and γ-butyrolactone are preferable, and tetrahydrofuran is more preferable.

Examples of sulfur-containing heterocyclic compound solvents include sulfolane and tetrahydrothiophene.

Examples of solvents that are heterocyclic compounds having two or more atoms among nitrogen, oxygen and sulfur include morpholine, N-methylmorpholine, thiazole, isothiazole, oxazole and benzoxazole.

As the amount of a solvent to be added that inhibits the dissolution of the protein of interest to the first aprotic polar solvent, the optimum amount may be determined according to the amount of an inorganic salt to be added, and, for example, with respect to the first aprotic polar solvent, may be 10 to 75% (vol/vol) and may be 10 to 50%.

The conditions under which the protein derived from the host cell dissolves but the recombinant protein does not dissolve can be set appropriately according to the concentration of the inorganic salt to be added to the first aprotic polar solvent and the recombinant protein of interest, but it is preferable that the conditions be a temperature condition. It is preferable to warm to a temperature at which the protein derived from the host cell dissolves but the recombinant protein of interest does not dissolve and to maintain for a predetermined time.

The temperature for dissolving may be determined according to the concentration of the inorganic salt to be added to the first aprotic polar solvent and the recombinant protein of interest, but examples thereof include temperatures of 10 to 60°C and 10 to 40°C. For example, the upper limit of the temperature for dissolving may be 70°C, 60°C, 50°C or 40°C, and the lower limit of the temperature for dissolving may be 10°C, 20°C, 25°C or 30°C.

The time for dissolving is not required to be particularly limited as long as the protein derived from the host cell is sufficiently dissolved and the recombinant protein of interest is dissolved in a small amount, but in consideration of industrial production, 10 to 100 min is preferable, 10 to 60 min is more preferable, and 10 to 30 min is further preferable.

When the recombinant protein of interest is a structural protein such as spider silk, silkworm silk, collagen, elastin and resilin, the following conditions can be mentioned, for example.

The amount of the first aprotic polar solvent to be added to the host expressing the structural protein is, per recombinant cell dry weight (wt), as the aprotic polar solvent (vol)/ recombinant cell dry weight (wt) ratio, preferably 10 to 100 times, more preferably 12 to 50 times, and further preferably 20 to 35 times. As the inorganic salt to be added to the first aprotic polar solvent, lithium chloride and calcium chloride are preferable, and lithium chloride is more preferable. In addition, the concentration at which the inorganic salt is added is preferably 0 to 1.0 M or less, more preferably 0 to 0.6 M or less and further preferably 0 to 0.5 M or less relative to the first aprotic polar solvent. By treating with the first aprotic polar solvent at, for example, 10 to 50°C, preferably 10 to 40°C, the protein constituting the host cell can be dissolved. The time for dissolving may be, for example, 20 to 60 min, preferably 20 to 40 min, and more preferably 20 to 30 min.

More specifically, when the recombinant protein of interest is a protein containing a domain sequence represented by Formula 1: [(A)ₙ motif-REP]ₘ, as in SEQ ID NOs:1 to 4, for example, the following conditions can be mentioned, wherein (A)ₙ motif represents an amino acid sequence composed of 4 to 20 amino acid residues and the number of alanine residues to the total number of amino acid residues in (A)ₙ motif is 80% or more; REP represents an amino acid sequence composed of 10 to 200 amino acid residues; and m represents an integer of 8 to 300; and a plurality of (A)ₙ motifs may be the same amino acid sequence or different amino acid sequences, and a plurality of REPs may be the same amino acid sequence or different amino acid sequences.

The amount of the first aprotic polar solvent to be added to the host expressing the protein is, per recombinant cell dry weight (wt), as the aprotic polar solvent (vol)/recombinant cell dry weight (wt) ratio, preferably 10 to 100 times, more preferably 12 to 50 times, further preferably 20 to 35 times. As the inorganic salt to be added to the first aprotic polar solvent, lithium chloride and calcium chloride are preferable, and lithium chloride is more preferable. In addition, the concentration at which the inorganic salt is added is preferably 0 to 1.0 M or less, more preferably 0 to 0.6 M or less and further preferably 0 to 0.5 M or less relative to the first aprotic polar solvent. By treating with the first aprotic polar solvent at, for example, 10 to 50°C, preferably 10 to 40°C, the protein constituting the host cell can be dissolved. The time for dissolving may be, for example, 20 to 60 min, preferably 20 to 40 min, and more preferably 20 to 30 min.

### [Step (B)]

The step (B) is a step of removing a first soluble fraction and recovering a first insoluble fraction containing the recombinant protein.

By removing the first soluble fraction in which the protein derived from the host cell is dissolved from the solution obtained in step (A), the protein derived from the host cell can be removed. Examples of methods for separating the first soluble fraction and the first insoluble fraction, removing the first soluble fraction in which the protein derived from the host cell is dissolved, and recovering the first insoluble fraction containing the recombinant protein of interest include general methods such as centrifugation, filter filtration such as drum filter and press filter. In the case of filter filtration, the first insoluble fraction containing the recombinant protein of interest can be recovered more efficiently by using both a filter aid such as celite and diatomaceous earth and a precoating agent.

According to this method, a protein derived from a host cell can be removed by separating a soluble fraction and an insoluble fraction by a simple method without adding an aqueous solvent such as water. Since no aqueous solvent is added, the first aprotic polar solvent used can be purified and used again for the step (A), which is economical.

### [Step (C)]

The step (C) is a step of treating the recovered first insoluble fraction using a second aprotic polar solvent to which an inorganic salt is added, under conditions where the recombinant protein dissolves.

The recombinant protein of interest contained in the first insoluble fraction recovered in step (B) can be effectively dissolved using an aprotic polar solvent (second aprotic polar solvent) to which an inorganic salt is added. By adding the second aprotic polar solvent to the first insoluble fraction and treating it under conditions where the recombinant protein dissolves, the recombinant protein dissolves and is contained in the soluble fraction (second soluble fraction), and insoluble contaminants are contained in the insoluble fraction (second insoluble fraction). By removing the second insoluble fraction in the following step (D), the contaminants can be removed.

As the second aprotic polar solvent, the aprotic polar solvent described in step (A) can be used, but it is preferable that it be an aprotic polar solvent having no cyclic structure, and it is more preferable that it be an aprotic polar solvent having no cyclic structure and having a dipole moment of 3.5 D or more.

As the second aprotic polar solvent, the same aprotic polar solvent as the first aprotic polar solvent used in step (A) may be used, or an aprotic polar solvent different from the first aprotic polar solvent may be used.

Preferable specific examples of the second aprotic polar solvent include DMSO, DMF, DMA, DM1 and NMP, and more preferable specific examples include DMSO, DMF and DMA.

The purity of the second aprotic polar solvent is not particularly limited, but from the viewpoint of reducing contaminants, it is preferable that the purity be 90% or more, and more preferable that it be 95% or more.

As the second aprotic polar solvent, one type of aprotic polar solvent may be used alone, or two or more types of aprotic polar solvents may be suitably mixed and used.

The amount of the second aprotic polar solvent to be added to the first insoluble fraction containing the recombinant protein of interest is, as the aprotic polar solvent (vol)/ recombinant cell dry weight (wt) ratio, preferably 5 to 50 times, more preferably 6 to 25 times, and further preferably 8 to 16 times.

As the inorganic salt to be added to the second aprotic polar solvent, the inorganic salts described in step (A) can be used. When the first aprotic polar solvent to which an inorganic salt is added is used in step (A), an inorganic salt of the same kind as that inorganic salt may be used as the inorganic salt to be added to the second aprotic polar solvent, or an inorganic salt different from that inorganic salt may be used.

As the inorganic salt to be added to the second aprotic polar solvent, one inorganic salt may be used alone, or two or more inorganic salts may be used in combination.

Suitable examples of the inorganic salt to be added to the second aprotic polar solvent include alkali metal halides and alkaline earth metal halides, and specific suitable examples include lithium chloride and calcium chloride.

As the amount of inorganic salt to be added to the second aprotic polar solvent, the optimum amount may be determined according to the second aprotic polar solvent to be used, and for example, an inorganic salt of 0.01 to 2.0 M to the second aprotic polar solvent can be added.

When DMSO is used as the second aprotic polar solvent, the amount of the inorganic salt to be added is preferably 0.01 to 1.5 M, more preferably 0.5 to 1.5 M, and further preferably 1 to 1.5 M.

When DMF, DMA, DMI and NMP are used as the second aprotic polar solvent, the amount of the inorganic salt to be added is preferably 0.01 to 2.0 M, more preferably 0.5 to 2.0 M and further preferably 1.0 to 2.0 M.

The conditions under which the recombinant protein dissolves can be set appropriately according to the concentration of the inorganic salt to be added to the second aprotic polar solvent and the recombinant protein of interest, but it is preferable that the conditions be a temperature condition. It is preferable to warm to a temperature at which the recombinant protein dissolves and to maintain for a predetermined time. Warming allows to efficiently dissolve the recombinant protein of interest.

The temperature for dissolving may be determined according to the concentration of the inorganic salt to be added and the recombinant protein of interest, but examples include temperatures of 10 to 90°C, preferably 30 to 85°C and more preferably 40 to 80°C. For example, the upper limit of the temperature for dissolving may be 100°C, 90°C, 85°C or 80°C, and the lower limit of the temperature for dissolving may be 50°C, 60°C, 65°C or 70°C.

The time for dissolving is not required to be particularly limited as long as the recombinant protein of interest sufficiently dissolves, but in consideration of industrial productivity and yield, 20 to 80 min is preferable, 20 to 70 min is more preferable, and 20 to 60 min is further preferable.

### [Step (D)]

The step (D) is a step of removing a second insoluble fraction to obtain a second soluble fraction containing the recombinant protein.

By removing the second insoluble fraction containing insoluble contaminants from the solution obtained in step (C), the contaminants can be removed. Example of methods for separating the second soluble fraction and the second insoluble fraction, removing the second insoluble fraction containing the insoluble contaminants and recovering the second soluble fraction containing the recombinant protein of interest include general methods such as centrifugation, filter filtration such as drum filter and press filter. In the case of filter filtration, the second soluble fraction in which the recombinant protein of interest is dissolved can be recovered more efficiently by using both a filter aid such as celite and diatomaceous earth and a precoating agent.

### [Step (E)]

The present purification method can further comprise the step (E) which treats the second soluble fraction using a poor solvent for the recombinant protein, thereby aggregating the recombinant protein and recovering the purified recombinant protein as an aggregate.

A solvent which hardly dissolves the recombinant protein (poor solvent) is added to the second soluble fraction in which the recombinant protein of interest obtained in the step (D) is dissolved, thereby aggregating the recombinant protein (poor solvent addition aggregation) to recover it as an aggregate, allows to obtain a recombinant protein having a further higher refined purity.

As the poor solvent, a solvent which makes it difficult for the recombinant protein of interest to be dissolved in the second aprotic polar solvent is preferable. Examples of poor solvents include esters, ketones, alcohols, nitriles, ethers and aromatic hydrocarbons.

Examples of poor solvents of esters include ethyl acetate, pentyl acetate, isopentyl acetate, octyl acetate, ethyl formate, ethyl butyrate, ethyl caproate, 2-ethoxyethyl acetate, methyl butyrate and methyl salicylate, and ethyl acetate, ethyl formate, ethyl butyrate and 2-ethoxyethyl acetate are preferable, and ethyl acetate and 2-ethoxyethyl acetate are more preferable.

Examples of poor solvents of ketones include acetone, methyl ethyl ketone, methyl butyl ketone and methyl isobutyl ketone, and acetone and methyl ethyl ketone are preferable, and acetone is more preferable.

As poor solvents of alcohols, saturated ones are preferable, and monohydric alcohols having 1 to 5 carbon atoms, dihydric alcohols having 2 to 5 carbon atoms and trihydric alcohols having 3 carbon atoms are more preferable.

Specific examples of monohydric alcohols include methanol, ethanol, 1-propanol, 2-propanol, 1-butanol, 2-butanol, isobutyl alcohol, 1-pentanol, 2- pentanol and 3-pentanol. Examples of dihydric alcohols include 1,2-ethanediol, 1,2-propanediol, 1,3-propanediol, 1,2-butanediol, 1,3-butanediol, 1,4-butanediol, 2,3-butanediol and 1,5-pentanediol. Examples of trihydric alcohols include glycerin and the like.

As poor solvents of nitriles, saturated ones are preferable, those with 2 to 8 carbon atoms, especially those with 2 to 6 carbon atoms are more preferable, and those with 2 to 4 carbon atoms are further preferable. Specific examples thereof include acetonitrile, propionitrile, succinonitrile, butyronitrile and isobutyronitrile.

As poor solvents of ethers, saturated ones are preferably used. Specific examples thereof include diethyl ether, methyl tert-butyl ether, anisole, dioxane and tetrahydrofuran.

Examples of poor solvents of aromatic hydrocarbons include benzene and toluene, and toluene is preferable.

The amount of the poor solvent to be added to the second soluble fraction may be appropriately determined according to the recombinant protein of interest, but usually the same amount as the second soluble fraction that dissolves the protein may be added. The amount to be added may be appropriately adjusted according to the state of aggregation of the protein and the presence of contamination of protein derived from the host cell.

Examples of methods for recovering the aggregated recombinant protein of interest as an aggregate include general methods such as centrifugation, filter filtration such as a drum filter or a press filter. In the case of filter filtration, a recombinant protein of interest can be recovered as an aggregate more efficiently by using both a filter aid such as celite and diatomaceous earth and a precoating agent.

According to this method, a recombinant protein of interest can be purified without adding an aqueous solvent such as water. Therefore, after recovering the recombinant protein as an aggregate, the second aprotic polar solvent used can be purified and reused, which is economical.

### [Method for Producing Artificial Polypeptide Fiber]

When the recombinant protein of interest is a protein derived from a structural protein produced for the purpose of producing a polypeptide fiber, such as spider silk or silkworm silk, the second soluble fraction containing the recombinant protein obtained in step (D) can be used as it is for the production of polypeptide fibers. Here, "used as it is" means that no further purification step is required. This indicates that the purity of the purified recombinant protein contained in the second soluble fraction obtained in step (D) is high enough to be used for spinning. After the steps (A) to (D), carrying out a spinning step allows to produce artificial polypeptide fibers by wet spinning.

That is, the method for producing artificial polypeptide fibers according to one embodiment comprises the following steps (A) to (D) and (F):
(A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
(B) a step of removing a first soluble fraction and recovering a first insoluble fraction containing the recombinant protein;
(C) a step of treating the recovered first insoluble fraction using a second aprotic polar solvent to which an inorganic salt is added, under conditions where the recombinant protein dissolves;
(D) a step of removing a second insoluble fraction to obtain a second soluble fraction containing the recombinant protein; and
(F) a step of applying the second soluble fraction as a filamentous liquid to a coagulation liquid and coagulating the recombinant protein in filament form and recovering the recombinant protein to obtain an undrawn yarn.

The steps (A) to (D) are as described above. The step (F) is a step of applying a second soluble fraction as a filamentous liquid to a coagulation liquid, thereby coagulating the recombinant protein in filament form, and recovering the recombinant protein to obtain an undrawn yarn.

When the second soluble fraction containing the recombinant protein obtained in step (D) is applied to the coagulation liquid, the recombinant protein coagulates. At this point, applying the second soluble fraction as a filamentous liquid to the coagulation liquid allows to coagulate the recombinant protein in filament form and to form a yarn (undrawn yarn). The formation of the undrawn yarn can be carried out, for example, according to the method described in Japanese Patent No. 5584932.
(1) Preparation of Polypeptide Solution (Dope Solution) As described above, the second soluble fraction in which the recombinant protein obtained in step (D) is dissolved does not require a further purification step and can be used as a so-called dope solution for spinning in step (E). The viscosity suitable for spinning is generally 10 to 10,000 cP (centipoise), and the viscosity can be measured using, for example, the product name "EMS viscometer" manufactured by Kyoto Electronics Industry Co., Ltd. If the viscosity of the second soluble fraction obtained in step (D) is not within the range of 10 to 10,000 cP (centipoise), the viscosity of the second soluble fraction may be adjusted to a spinnable viscosity. For adjusting the viscosity, the aprotic polar solvents exemplified as suitable solvents in the explanation of step (A) can be used. The aprotic polar solvent may contain a suitable inorganic salt exemplified in the explanation of step (A).

In order to use it as a dope solution, the concentration of the inorganic salt to be added to the aprotic polar solvent when adjusting the viscosity may be higher than that for the purpose of purification. For example, an inorganic salt may be added in an amount of 0.5 to 2.0 M to the dope solution.

Whether the second soluble fraction can actually be used as it is for spinning in step (E) as a dope solution can be confirmed, for example, by the following simple method. That is, an appropriate amount of the second soluble fraction is placed in a syringe having a needle with an inner diameter of 0.1 mm, the tip of the needle is immersed in methanol (corresponding to the coagulation liquid) in a 300 mL beaker and extruded. When the recombinant protein coagulates as a yarn in methanol and can be removed from the methanol as a yarn with tweezers, the second soluble fraction can be used as a dope solution for spinning. When a yarn is not formed, it can be used as a dope solution by reviewing the viscosity, the inorganic salt concentration and the like and finely adjusting them.

### (2) Polymerization of Polypeptide Using Polypeptide Solution

The solution of the polypeptide prepared in (1) (dope solution) is heated to 100°C or more. The heating causes dehydration condensation between the polypeptides and polymerization occurs. Using a known dehydration condensation catalyst in combination allows to dramatically increase the polymerization efficiency. The dope solution subjected to the polymerization reaction can be diluted by adding ethyl alcohol, methyl alcohol, water or the like as necessary, and can be used for production of wet spinning.

### (3) Wet spinning - Drawing

### (a) Wet spinning

The second soluble fraction (dope solution) after heating is applied to a coagulation liquid as a filamentous fluid. The coagulation liquid to be used for wet spinning may be any solution as long as it can remove the solvent. It is preferable to use a lower alcohol having 1 to 5 carbon atoms such as methanol, ethanol and 2-propanol or acetone as the coagulation liquid for desorbing the solvent and forming fibers. Water may be added as appropriate. From the viewpoint of the stability of spinning, the temperature of the coagulation liquid is preferably 5 to 30°C.

The method for applying the second soluble fraction (dope solution) as a filamentous fluid is not particularly limited, but an example thereof include the method of extruding from a spinneret into the coagulation liquid of a desolvation tank. An undrawn yarn is obtained by the coagulation of the recombinant protein. The extrusion rate in the case of extruding the second soluble fraction into the coagulation liquid can be appropriately set according to the diameter of the spinneret and the viscosity of the second soluble fraction, but, for example, in the case of a syringe pump having a nozzle with a diameter of 0.1 to 0.6 mm, from the viewpoint of the stability of spinning, the extrusion rate is preferably 0.2 to 6.0 mL/h per hole and more preferably 1.4 to 4.0 mL/h per hole. The length of the desolvation tank (coagulation liquid tank) into which the coagulation liquid is placed is not particularly limited, but may be, for example, 200 to 500 mm. The take-off speed of the undrawn yarn formed by the coagulation of the recombinant protein may be, for example, 1 to 14 m/min, and the residence time may be, for example, 0.01 to 0.15 min. From the viewpoint of desolvation efficiency, the take-off speed of the undrawn yarn is preferably 1 to 3 m/min. The undrawn yarn formed by the coagulation of the recombinant protein may further be drawn (pre-drawn) in the coagulation liquid, but from the viewpoint of suppressing the evaporation of the lower alcohol used for the coagulation liquid, it is preferable that the coagulation liquid be maintained at a low temperature and taken off from the coagulation liquid in the state of undrawn yarn.

### (b) Drawing

The method for producing an artificial polypeptide fiber according to the present embodiment may also include a step of further drawing the undrawn yarn obtained in step (E). The drawing may be a single-stage drawing or a multistage drawing of two or more stages. When stretched in multiple stages, since molecules can be oriented in multiple stages and the total draw ratio can also be increased, it is suitable for producing fibers with high toughness.

### [Method for Producing Polypeptide Film]

When the recombinant protein of interest is a protein derived from a structural protein produced for the purpose of producing a polypeptide film, such as spider silk and silkworm silk, the second soluble fraction containing the recombinant protein obtained in step (D) can be used as it is for the production of a polypeptide film. Here, "used as it is" means that no further purification step is required. This indicates that the purity of the purified recombinant protein contained in the second soluble fraction obtained in step (D) is high enough to be used for the formation of a polypeptide film. After the steps (A) to (D), carrying out a film formation step allows to produce a polypeptide film.

That is, the method for producing a polypeptide film according to one embodiment comprises the following steps (A) to (D) and (G):
(A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
(B) a step of removing a first soluble fraction and recovering a first insoluble fraction containing the recombinant protein;
(C) a step of treating the recovered first insoluble fraction using a second aprotic polar solvent to which an inorganic salt is added, under conditions where the recombinant protein dissolves;
(D) a step of removing a second insoluble fraction to obtain a second soluble fraction containing the recombinant protein; and
(G) a step of forming a coating film of a predetermined thickness on a flat plate resistant to the second aprotic polar solvent using the second soluble fraction, and removing the second aprotic polar solvent from the coating film to obtain a polypeptide film.

The steps (A) to (D) are as described above. The step (G) is a step of forming a coating film of a predetermined thickness on a flat plate resistant to the second aprotic polar solvent using the second soluble fraction, and removing the second aprotic polar solvent from the coating film to obtain a polypeptide film.

The polypeptide film of the recombinant protein is formed when a coating film is formed using the second soluble fraction containing the recombinant protein of interest obtained in step (D) and the second aprotic polar solvent contained in the second soluble fraction is removed.

As the flat plate forming the coating film, a flat plate resistant to the second aprotic polar solvent such as a glass plate is used. The thickness of the coating film is not particularly limited, but may be, for example, 1 to 1000 µm.

An example of the method for forming a polypeptide film having a predetermined thickness is the casting method. When forming a polypeptide film by the casting method, the polypeptide film can be obtained by casting the second soluble fraction containing the recombinant protein of interest on a flat plate to a thickness of several microns or more using a tool such as a doctor coat or a knife coater to form a cast film, and then desorbing the solvent by drying under reduced pressure or immersion in a desolvation tank.

### Examples

Hereinafter, the present invention will be described in more detail based on Examples. However, the present invention is not limited to the following Examples.

### (1) Preparation of a protein of interest-expressing strain (recombinant cell)

The nucleic acids GEN514, GEN740, GEN797 and GEN 796, encoding fibroins having a sequence derived from spider silk having an amino acid sequence represented by SEQ ID NO:1 (PRT468), SEQ ID NO:2 (PRT647), SEQ ID NO:3 (PRT699) and SEQ ID NO:4 (PRT698), respectively, were synthesized. To the nucleic acids, an NdeI site was added to the 5' terminus and an EcoRI site was added downstream of the stop codon. The hydropathy index and molecular weight of each protein are as shown in Table 2.

**[Table 2]**

| SEQ ID NO: | Protein name | Nucleic acid name | Hydropathy index | Molecular weight |
|---|---|---|---|---|
| 1 | PRT468 | GEN514 | -0.59 | 50.1 |
| 2 | PRT647 | GEN740 | 0.04 | 54.1 |
| 3 | PRT699 | GEN797 | 0.17 | 48.8 |
| 4 | PRT698 | GEN796 | 0.43 | 48.5 |

These four nucleic acids were each cloned into a cloning vector (pUC118). Thereafter, the nucleic acids were treated with a restriction enzyme at Ndel and EcoRI and excised, and then recombined into the protein expression vector pET-22b (+) to obtain an expression vector. Transformed Escherichia coli (recombinant cell) expressing the protein of interest was obtained by transforming Escherichia coli BLR (DE3) with each pET-22b (+) expression vector in which each of the four nucleic acids was recombined.

### (2) Expression of the protein of interest

The transformed Escherichia coli was cultured in 2 mL of LB medium containing ampicillin for 15 h. The culture solution was added to 100 mL of seed culture medium containing ampicillin (Table 3) so that the OD₆₀₀ be 0.005. The culture solution temperature was kept at 30°C and flask cultivation was carried out until the OD₆₀₀ reached 5 (about 15 h) to obtain a seed culture solution.

**[Table 3]**

| Seed culture medium | |
|---|---|
| Reagent | Concentration (g/L) |
| Glucose | 5.0 |
| KH₂PO₄ | 4.0 |
| K₂HPO₄ | 10.0 |
| Yeast extract | 6.0 |
| Ampicillin | 0.1 |

The seed culture solution was added to a jar fermenter to which 500 mL of production medium (Table 4) had been added so that the OD₆₀₀ be 0.05. The culture solution temperature was kept at 37°C, and the culture was carried out with constant control at pH 6.9. In addition, the dissolved oxygen concentration in the culture solution was maintained at 20% of the dissolved oxygen saturation concentration.

**[Table 4]**

| Production medium | |
|---|---|
| Reagent | Concentration (g/L) |
| Glucose | 12.0 |
| KH₂PO₄ | 9.0 |
| MgSO₄•7 H₂O | 2.4 |
| Yeast extract | 15 |
| FeSO₄•7 H₂O | 0.04 |
| MnSO₄•5 H₂O | 0.04 |
| CaCl₂•2 H₂O | 0.04 |
| ADEKA NOL LG-295S (Antifoaming agent) | 0.1 (mL/L) |

Immediately after the glucose in the production medium was completely consumed, a feed solution (455 g/1L of glucose, 120 g/1L of yeast extract) was added at a rate of 1 mL/min. The culture solution temperature was kept at 37°C, and the culture was carried out with constant control at pH 6.9. In addition, the dissolved oxygen concentration in the culture solution was maintained at 20% of the dissolved oxygen saturation concentration and the culture was carried out for 20 h. Thereafter, 1 M of isopropyl-β-thiogalactopyranoside (IPTG) was added so that the final concentration be 1 mM to the culture solution and the expression of the protein of interest was induced. Twenty hours after the addition of IPTG, the culture solution was centrifuged to recover the bacterial cells. SDS-PAGE was performed using the bacterial cells prepared from the culture solution before IPTG addition and after IPTG addition, and it was confirmed that the protein of interest was expressed as an insoluble matter by the appearance of a band of a protein-of-interest size depending on IPTG addition.

### [Example 1 Influence of Inorganic Salt and Temperature on the Dissolving Step of Host Cell-Derived Protein]

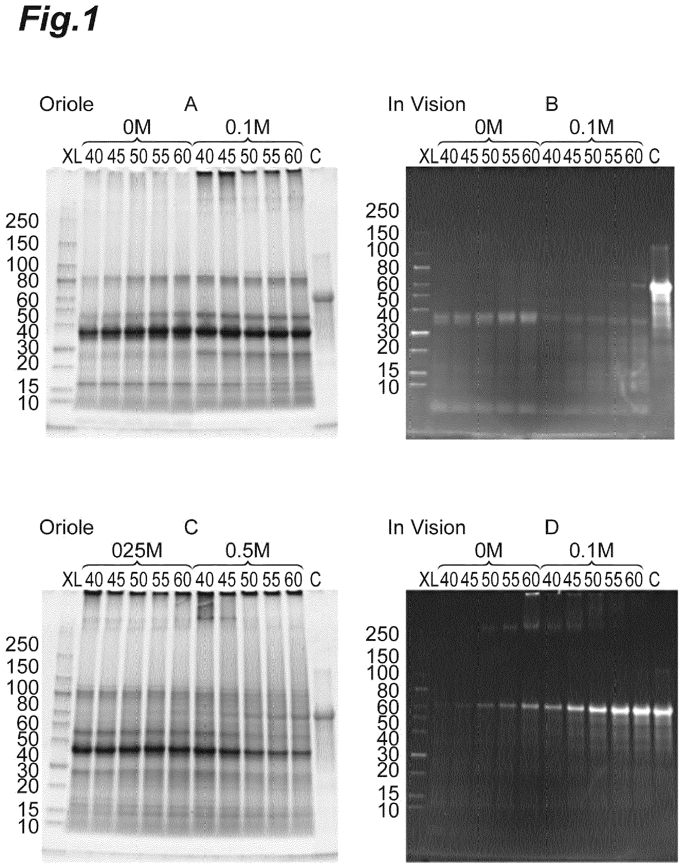

The influence of the addition of an inorganic salt to the first aprotic polar solvent and the addition concentration, as well as the temperature in the step of dissolving the protein derived from the host cell was investigated. 1 ml of DMSO containing 0, 0.1, 0.25 and 0.5 M lithium chloride was added to 50 mg of dried bacterial cells of PRT468 expressing Escherichia coli and treated at the temperatures of 40, 45, 50, 55 and 60°C for 30 min. After cooling to room temperature, centrifugation was performed under the conditions of 11,000 × g and 5 min. The supernatant obtained by centrifugation (first soluble fraction) was analyzed by SDS-PAGE. The results are shown in Figure 1. The numbers from 40 to 60 indicate the temperature, and the notation of 0 to 0.5 M indicates the concentration of lithium chloride contained in DMSO. Lane XL is the lane where a molecular weight marker protein has migrated, and lane C where a purified PRT468 has migrated as a positive control.

Figures 1A and 1C have been stained by Oriole (trademark) fluorescent gel stain (manufactured by Bio-Rad) capable of staining all proteins and Figures 1B and ID by InVision (trademark) His-tagged in-gel staining reagent (manufactured by Thermo Fisher Scientific) reacting to the His tag region of PRT468.

In both conditions, a protein band derived from the host cell was confirmed, and it was shown that the protein derived from the host cell was dissolved in the first aprotic polar solvent and contained in the first soluble fraction. However, in the sample using DMSO containing a high concentration of lithium chloride of 0.25 M to 0.5 M, the protein of interest PRT468 was also dissolved and was contained in the first soluble fraction (Figure 1D). The conditions under which the protein derived from the host cell dissolves and the dissolution of PRT468 is the lowest were the conditions where 0.1 M of lithium chloride was added to DMSO and treated at 40°C.

### [Example 2 First effect of the addition of cyclohexanone in the dissolution step of the protein derived from the host cell]

In Example 1, when DMSO containing a high concentration of lithium chloride was used as the first aprotic polar solvent, not only the protein derived from the host cell but also the protein of interest PRT468 was dissolved. Therefore, the effect on preventing the dissolution of PRT468 by adding cyclohexanone to DMSO containing a high concentration of lithium chloride was examined.

Solvents with three mixing ratios of DMSO: cyclohexanone = 100:0, 50:50 and 25:75 were prepared, and lithium chloride was added to these solvents to be 0.1 or 0.5 M, and a total of 6 types of first aprotic polar solvent were prepared (see Table 5).

**[Table 5]**

| Lane No. | Lithium chloride (M) | DMSO (%) | Cyclohexanone (%) |
|---|---|---|---|
| 1 | 0.1 | 100 | 0 |
| 2 | 0.1 | 50 | 50 |
| 3 | 0.1 | 25 | 75 |
| 4 | 0.5 | 100 | 0 |
| 5 | 0.5 | 50 | 50 |
| 6 | 0.5 | 25 | 75 |

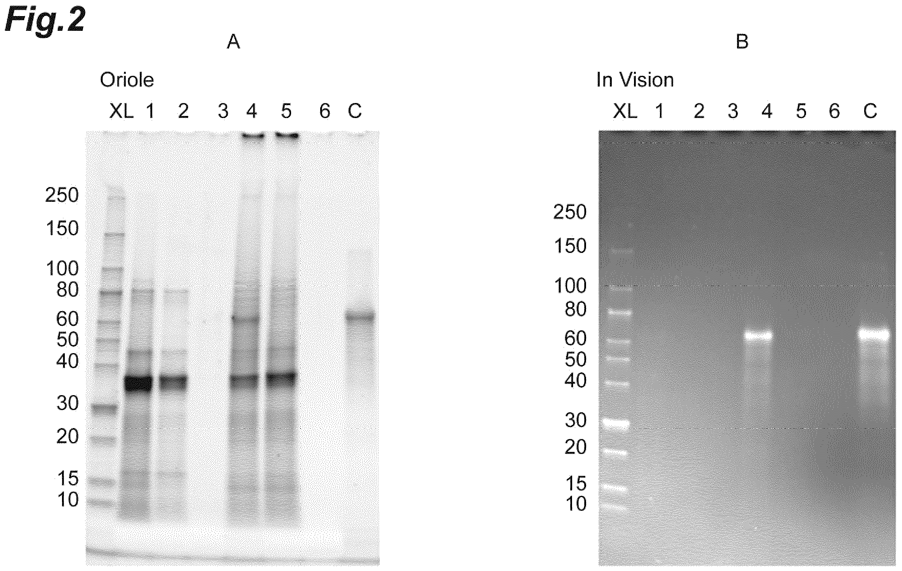

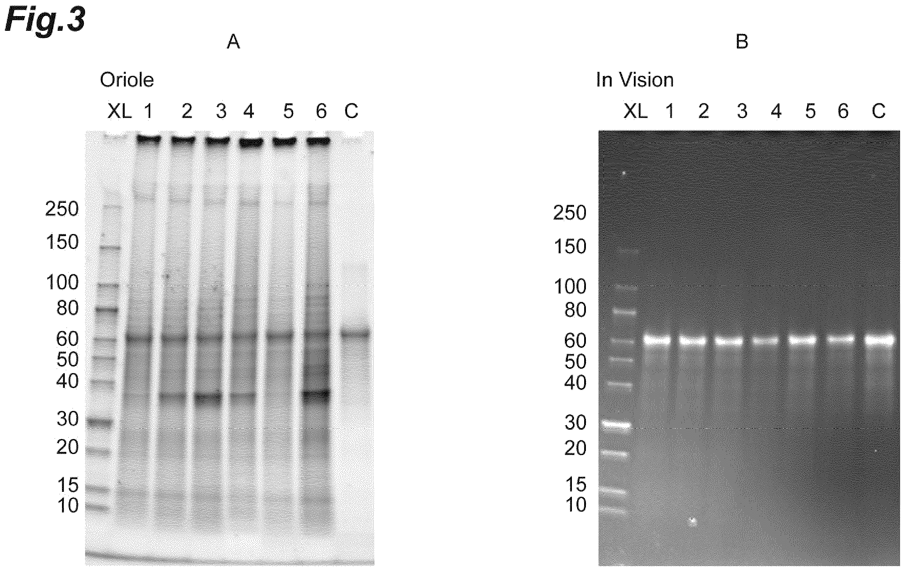

1 mL of the first aprotic polar solvents shown in Table 5 was added to 50 mg of dried bacterial cells of PRT468-expressing Escherichia coli and treated at 60°C for 30 min. After cooling to room temperature, centrifugation was performed under the conditions of 11,000 × g and 5 min. 0.5 mL of the treatment solvent used were each added to the precipitate obtained by centrifugation and suspended, then centrifuged again, followed by washing of the precipitate fraction. The obtained supernatant fraction (first soluble fraction) and precipitate fraction (first insoluble fraction) were analyzed by SDS-PAGE. The result of the supernatant fraction is shown in Figure 2, and the result of the precipitate fraction is shown in Figure 3. The numbers 1 to 6 correspond to the lane numbers in Table 5. Lane XL is the lane where a molecular weight marker protein has migrated, and lane C where a purified PRT468 has migrated as a positive control.

Figures 2A and 3A have been stained by Oriole (trademark) fluorescent gel stain capable of staining all proteins and Figures 2B and 3B by In Vision (trademark) His-tagged in-gel staining reagent reacting to the His tag region of PRT468.

When DMSO containing 0.5 M lithium chloride without added cyclohexanone was used as the first aprotic polar solvent, a band was confirmed at the same position as the positive control and the dissolution of PRT468 was observed (Lane 4 of Figures 2A and 2B). On the other hand, when DMSO containing 0.5 M lithium chloride to which cyclohexanone was added was used as the first aprotic polar solvent, a band of PRT468 was not confirmed. (Lanes 5 and 6 of Figures 2A and 2B).

When DMSO containing 0.5 M lithium chloride was used, a decrease in PRT468 contained in the precipitate fraction was observed (Lane 4 in Figures 3A and 3B), compared to when DMSO containing 0.1 M lithium chloride was used (Lane 1 in Figures 3A and 3B). However, when cyclohexanone was added, there was no decrease observed in the content of PRT468 in the precipitate fraction (Lanes 5 and 6 in Figures 3A and 3B). However, when a first aprotic polar solvent having DMSO:cyclohexanone of 25:75 was used, it resulted in the protein derived from the host cell remaining in a large amount in the precipitate fraction together with PRT468 (Lane 6 in Figure 3A), since the PRT468 contained in the supernatant fraction decreased, but the protein derived from the host cell contained in the supernatant fraction also decreased, and these were not suitable conditions for the purification of PRT468.

From these results, after confirming the solubility of the protein of interest in the first aprotic polar solvent, it was confirmed that adding cyclohexanone at an appropriate concentration according to the protein allowed to make the protein derived from the host cell more soluble in the first aprotic polar solvent by adding a high concentration of a salt, while preventing the dissolution of the protein of interest in the first aprotic polar solvent. Thus, it is possible to efficiently separate the protein derived from the host cell into the first soluble fraction and the protein of interest into the first insoluble fraction.

### [Example 3 Influence of the Type of Aprotic Polar Solvent in the Step of Dissolving the Protein Derived from Host Cell]

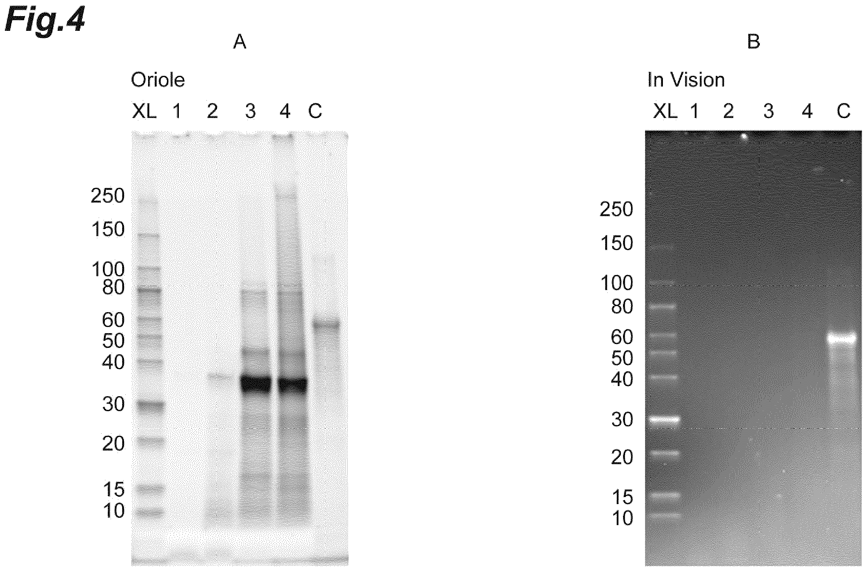

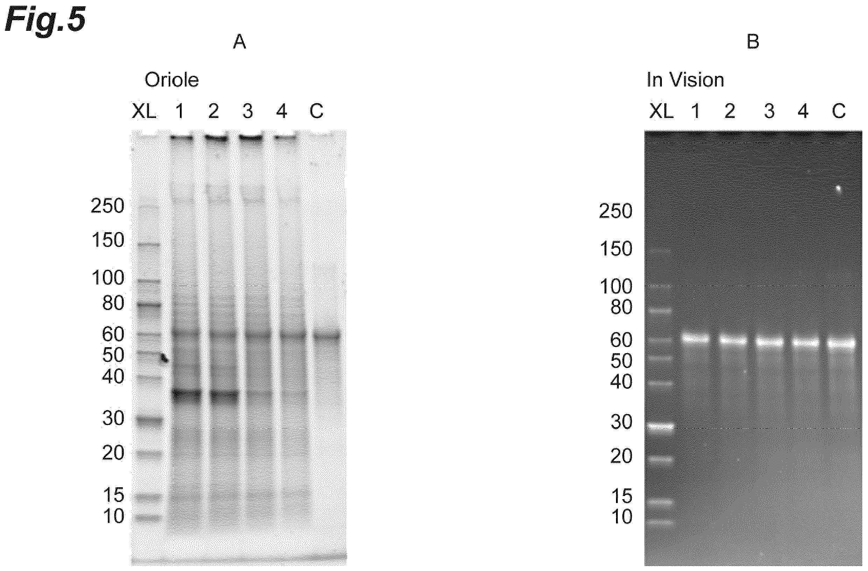

It was examined whether the protein derived from the host cell and the protein of interest can be efficiently separated when DMF was used as the first aprotic polar solvent. 1 mL of DMF (first aprotic polar solvent) containing 0, 0.1, 0.25 and 0.5 M lithium chloride was added to 50 mg of dried bacterial cells of PRT468-expressing Escherichia coli and treated at a temperature of 60°C for 30 min. After cooling to room temperature, centrifugation was performed at 11,000 × g for 5 min. 0.5mL of the first aprotic polar solvent used were each added to the precipitate fraction obtained by centrifugation and suspended, then centrifuged again, followed by washing of the precipitate fraction. The obtained supernatant fraction (first soluble fraction) and precipitate fraction (first insoluble fraction) were analyzed by SDS-PAGE. The result of the supernatant fraction is shown in Figure 4, and the result of the precipitate fraction is shown in Figure 5. Lanes 1 to 4 show the results that the concentrations of lithium chloride contained in DMF are 0 (Lane 1), 0.1 (Lane 2), 0.25 (Lane 3) and 0.5 M (Lane 4). Lane XL is the lane where a molecular weight marker protein has migrated, and lane C where a purified PRT468 has migrated as a positive control.

Figures 4A and 5A have been stained by Oriole (trademark) fluorescent gel stain capable of staining all proteins and Figures 4B and 5B by In Vision (trademark) His-tagged in-gel staining reagent reacting to the His tag region of PRT468.

The supernatant fraction contained more protein derived from the host cell in the case where DMF to which lithium chloride had been added was used as the first aprotic polar solvent, rather than DMF to which no lithium chloride had been added. Especially, when DMF containing 0.25 M or more of lithium chloride was used, it was shown that the protein derived from the host cell could be effectively dissolved (Figure 4A). In both cases, the supernatant fraction contained almost no PRT468 (Figure 4B).

When DMF containing 0.25 M or more lithium chloride was used as the first aprotic polar solvent, it was shown that proteins other than PRT468 contained in the precipitate fraction were remarkably few and that the protein derived from the host cell could be effectively removed, compared to when DMF containing 0 to 0.1 lithium chloride was used (Figure 5A). In addition, it was also shown that PRT468 was less dissolved and that it could be recovered without loss (Figure 5B). This allowed to confirm that, similarly to DMSO, optimizing the concentration of lithium chloride makes it possible to also use DMF as the first aprotic polar solvent for removing the proteins derived from the host cells.

### [Example 4 Removal of Protein Derived from Host Cell and Purification of the Protein of Interest]

The proteins derived from the host cells were removed from the transformed Escherichia coli expressing PRT468 (SEQ ID NO:1: molecular weight 50.1, HI: -0.59), PRT647 (SEQ ID NO:2: molecular weight 54.1, HI: 0.04), PRT699 (SEQ ID NO:3: molecular weight 48.8, HI: 0.17) and PRT698 (SEQ ID NO:4: molecular weight 48.5, HI: 0.43) respectively, and the purification of the recombinant proteins of interest was performed.

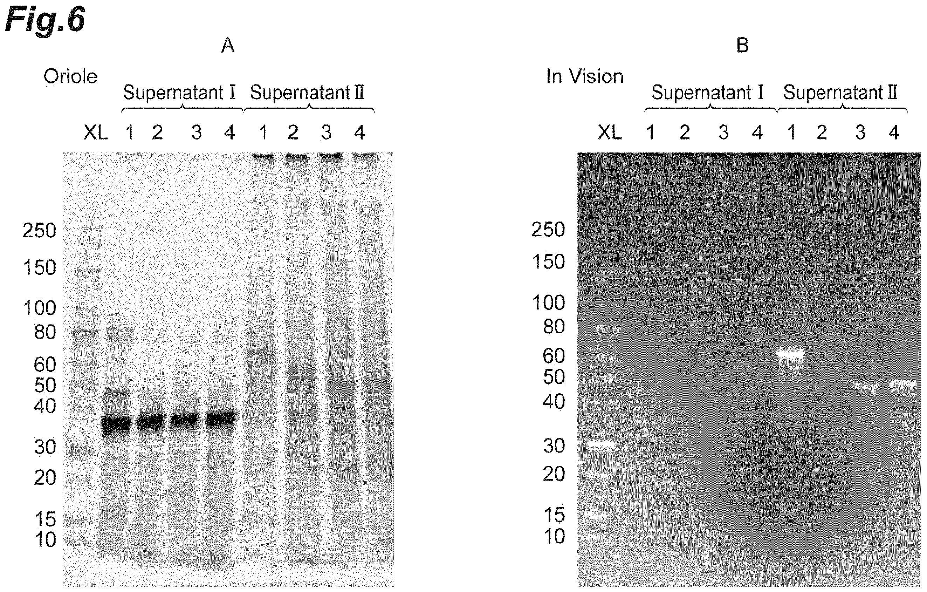

1 mL of DMSO containing 0.1 M lithium chloride (first aprotic polar solvent) was added to 50 mg of the dried bacterial cells of the transformed Escherichia coli expressing each of the above four proteins of interest, and was treated at a temperature of 40°C for 30 min. After cooling to room temperature, centrifugation was performed under the conditions of 11,000 × g and 5 min, and it was separated into the precipitate fraction (first insoluble fraction) and the supernatant fraction (supernatant I: first soluble fraction). For the precipitate fraction, 0.5 mL of DMSO containing 0.1 M lithium chloride was added again, suspended, and the precipitate fraction was washed by centrifugation. 0.5 mL of DMSO containing 0.5 M lithium chloride (second aprotic polar solvent) was added to the precipitate fraction after the washing, and was treated at 80°C for 30 min. After cooling to room temperature, centrifugation was performed under the conditions of 20,000 × g and 5 min, and the supernatant fraction containing the protein of interest (Supernatant II: second soluble fraction) was recovered. Supernatant I and Supernatant II were analyzed by SDS-PAGE. The results are shown in Figure 6. In Lanes 1 to 4, Supernatant I and Supernatant II were applied so that each protein concentration be 1.5 µg, and the results for PRT468 (Lane 1), PRT647 (Lane 2), PRT698 (Lane 3) and PRT699 (Lane 4) are shown. Lane XL is the lane where a molecular weight marker protein has migrated.

Figure 6A was stained by Oriole (trademark) fluorescent gel stain capable of staining all proteins and Figure 6B by In Vision (trademark) His-tagged in-gel staining reagent reacting to the His tag region of the amino acid sequence (His Tag sequence and hinge sequence) represented by SEQ ID NO:8 added to the N-terminus of the protein of interest.

The proteins derived from the host cells were efficiently removed in any of the four types of proteins (Figure 6A), and the proteins of interest could be purified (Figure 6B).

### [Example 5 Second Effect of Adding Cyclohexanone in the Step of Dissolving Protein Derived from Host Cell]

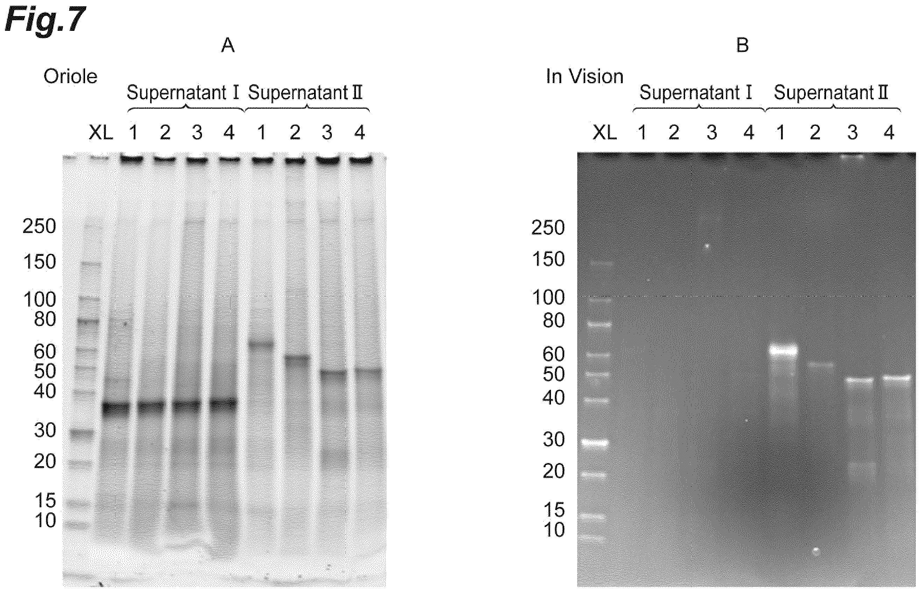

It was investigated whether the effect of preventing the dissolution of the protein of interest could be obtained by adding cyclohexanone to DMSO, even when the temperature for dissolving the protein derived from the host cell was raised. Except that the temperature in the step of dissolving the protein derived from the host cell was set to 40°C to 60°C, and that a solvent containing the same volume of cyclohexanone as DMSO and 0.5 M lithium chloride was used as the first aprotic polar solvent, the protein derived from the host cell was dissolved with the same method as in Example 4 and separated into a precipitate fraction (first insoluble fraction) and a supernatant fraction (Supernatant I: first soluble fraction). Furthermore, the precipitate fraction was washed with the same method as in Example 4, the protein of interest was subsequently dissolved, and the supernatant fraction containing the protein of interest (Supernatant II: second soluble fraction) was obtained. The results are shown in Figure 7. In Lanes 1 to 4, Supernatant I or Supernatant II were applied so that each protein concentration be 1.5 µg, and the results for PRT468 (Lane 1), PRT647 (Lane 2), PRT698 (Lane 3) and PRT 699 (Lane 4) are shown. Lane XL is the lane where a molecular weight marker protein has migrated.

Even when the temperature for dissolving the protein derived from the host cell was increased from 40°C to 60°C, the addition of cyclohexanone allowed to suppress the dissolution of the four proteins of interest and efficiently remove the proteins derived from the host cells (Supernatant I in Figures 7A and 7B). In addition, it was shown that the protein of interest could be purified with higher purity than in Example 4, since the bands other than the protein of interest were smaller or thinner compared to the results of Example 4 (Supernatant II of Figure 7A).

### [Example 6 First Effect of Purification Using Poor Solvent]

After obtaining the second soluble fraction containing the protein of interest, the effect of purification in the case of further purification using a poor solvent was examined. 200 mL of DMSO containing 0.1 M lithium chloride (first aprotic polar solvent) was added to 10 g of dried bacterial cells of transformed Escherichia coli expressing PRT468 and was treated at a temperature of 40°C for 1 h. 2 g of filtration aid Radiolite #900 was added to the treated solution, and after stirring, filtration was carried out under reduced pressure using a Buchner funnel equipped with a Glass Fiber Filter DP-7 (manufactured by ADVANTEC) to obtain a filtrate I (first soluble fraction). The residue filtered off (first insoluble fraction) was washed with 40 mL of DMSO containing 0.1 M lithium chloride. 200 mL of DMSO containing 0.5 M lithium chloride (second aprotic polar solvent) was added to the washing residue and treated at a temperature of 80°C for 1 h to dissolve PRT468. After cooling to room temperature, filtration under reduced pressure under the same conditions as above was performed to obtain a filtrate II (second soluble fraction) containing PRT468. This filtrate II was further purified using a poor solvent as follows.

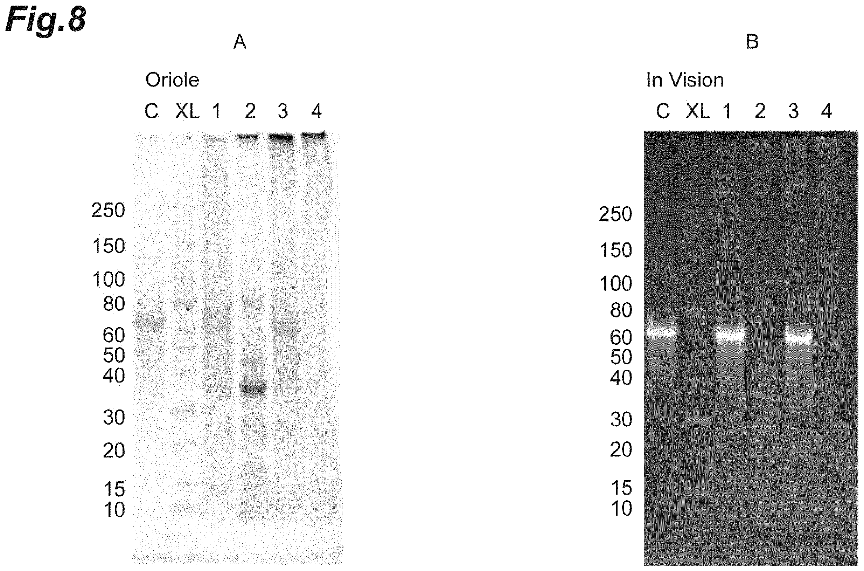

An equivalent amount of ethyl acetate (poor solvent) was added to 113 mL of filtrate II to aggregate PRT468. Centrifugation was performed with the conditions of 11,000 × g and 5 min to separate into a supernatant fraction (supernatant after centrifugation) and a precipitate fraction (aggregate). The precipitate fraction was recovered, washed three times with 200 mL of RO water and then freeze-dried to obtain 2.5 g of powder (purified powder). The purification state in each step was analyzed by SDS-PAGE. The results are shown in Figure 8. In Lane 1, the purified powder finally obtained, in Lane 2, the filtrate I, in Lane 3, the filtrate II, and in Lane 4, the supernatant after centrifugation were each applied so that each protein concentration be 1.5 µg. Lane XL is the lane where a molecular weight marker protein has migrated, and lane C where a purified PRT468 has migrated as a positive control.

It was confirmed that in the filtrate I after dissolving the protein derived from the host cell, a large amount of the protein derived from the host cell was dissolved and that it was possible to efficiently separate it from the filtered residue containing the protein (Lane 2 in Figure 8A). Moreover, it was confirmed that the protein of interest PRT468 had been effectively purified almost without loss by purification by addition of ethyl acetate, since no difference in the amount of PRT468 in the filtrate II prior to purification by addition of ethyl acetate and in the purified powder after purification was observed (Lanes 1 and 3 in Figure 8B).

### [Example 7: Second Effect of Purification Using Poor Solvent]

The effect of the combination of dissolution conditions of the host cell-derived protein and purification using a poor solvent was investigated in the method for purifying the proteins of interest from transformed Escherichia coli expressing each PRT468 (SEQ ID NO:1), PRT647 (SEQ ID NO:2), PRT699 (SEQ ID NO:3) and PRT 698 (SEQ ID NO:4).

Using transformed Escherichia coli expressing each of the above four proteins of interest, the dissolution treatment of the proteins derived from the host cells was performed with the same method as in Example 4 (40°C) or with the same method as in Example 5 (60°C-cyclohexanone addition). Thereafter, the washing of the precipitate fraction was performed in the same way as in Example 4, by adding 0.5 mL of DMSO containing 0.1 M lithium chloride to the precipitate fraction (first insoluble fraction) obtained by centrifugation, by suspending, and centrifuging. 0.5 mL of DMSO containing 0.5 M of lithium chloride (second aprotic polar solvent) was added to the precipitate fraction after the washing and was treated at 80°C for 30 min to dissolve the four proteins of interest. After cooling to room temperature, centrifugation was performed under the conditions of 20,000 × g and 5 min to obtain a supernatant fraction containing the proteins of interest (second soluble fraction).

600 µL of isopropanol (poor solvent) was added to 300 µL of the supernatant fraction, and mixed by inversion for 30 min to aggregate the proteins of interest. Centrifugation was performed with the conditions of 11,000 × g and 5 min to separate into a supernatant fraction (Supernatant I) and a precipitate fraction (aggregate). 1 mL of RO water was added to the precipitate fraction and washed. After performing centrifugation with the conditions of 11,000 × g and 5 min and removing the supernatant fraction (Supernatant II), the precipitate fraction was freeze-dried. 500 µL of DMSO containing 0.5 M lithium chloride was added to the dried precipitate fraction and treated at a temperature of 80°C for 30 min to obtain a solution containing the four purified proteins of interest (purified protein solution).

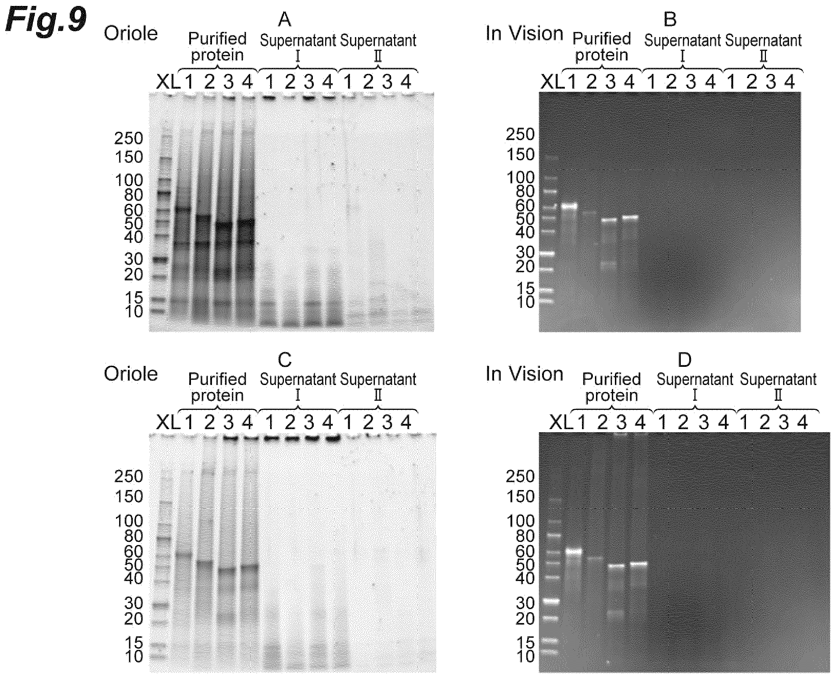

The solution of the four purified proteins of interest, Supernatant I and Supernatant II were analyzed by SDS-PAGE. Figures 9A and 9B show the results of performing the dissolution treatment of the protein derived from the host cell in the same way as in Example 4 (40°C), and Figures 9C and 9D show the results of performing the dissolution treatment of the protein derived from the host cell in the same way as in Example 5 (60°C-Cyclohexanone addition). In Lanes 1 to 4, the purified protein solution, supernatant I or supernatant II were applied so that each protein concentration be 1.5 µg, and the results for PRT468 (Lane 1), PRT647 (Lane 2), PRT698 (Lane 3) and PRT 699 (Lane 4) are shown.

Figures 9A and 9C were stained by Oriole (trademark) fluorescent gel stain capable of staining all proteins and Figures 9B and 9D by In Vision (trademark) His-tagged in-gel staining reagent reacting to the His tag region of the amino acid sequence (His Tag sequence and hinge sequence) represented by SEQ ID NO:8 added to the N-terminus of the protein of interest.

Compared to when combining the dissolution of the protein derived from the host cell at a temperature condition of 40°C with the aggregation by addition of the poor solvent isopropanol, combining the dissolution of the protein derived from the host cell under the conditions of 60°C-cyclohexanone addition with the aggregation by addition of the poor solvent isopropanol, decreased the bands other than the protein of interest in the protein after purification in any protein of interest, and further improvement in purity was observed ("Purified protein" in Figures 9A and 9C). On the other hand, the purification loss was almost equivalent when treating at a temperature condition of 40°C and at the conditions of 60°C-cyclohexanone addition (Figures 9B and 9D).

### [Example 8 Utilization of Second Soluble Fraction as Dope Solution]

Whether spinning is possible using the purified protein solution obtained by the present purification method was investigated.

According to the method described in Example 2, the host cell-derived protein was removed as follows. 200 mL of a first aprotic polar solvent, in which lithium chloride was added to be 0.5 M in a solvent mixed with the ratio DMSO:cyclohexanone = 1:1, was added to 10 g of dried bacterial cells of Escherichia coli expressing PRT468, and treated at a temperature of 60°C for 30 min. Next, centrifugation was performed under the conditions of 11,000 × g and 5 min. The supernatant fraction (first soluble fraction) was removed and the precipitate fraction (first insoluble fraction) was washed with 100 mL of the first aprotic polar solvent.

500 µL of DMSO containing 1.0 M lithium chloride (second aprotic polar solvent) was added to 500 mg of the precipitate fraction in a wet state and was treated at a temperature of 80°C for 30 min to dissolve the protein of interest. After cooling to room temperature, centrifugation was performed under the conditions of 20,000 × g and 60 min and the precipitate fraction (second soluble fraction) was removed to obtain the supernatant fraction (second soluble fraction) containing PRT468 as a purified protein solution. Whether the purified protein solution can be used as a dope solution for spinning was confirmed by the following method.

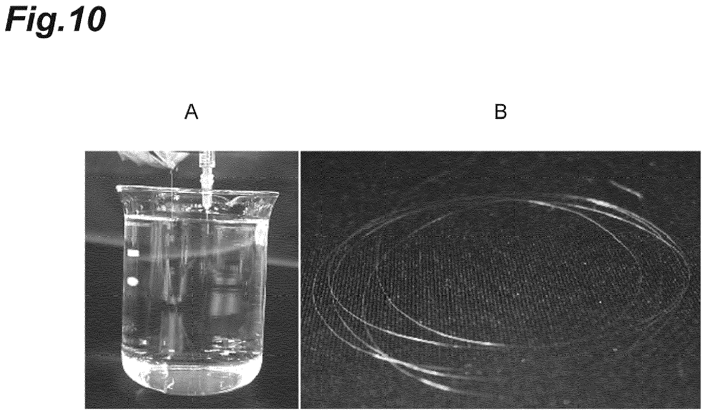

80 µL of the above purified protein solution containing PRT468 was placed in a syringe having a needle with an inner diameter of 0.1 mm. The tip of the needle of the syringe was immersed in methanol (coagulation liquid) in a 300 mL beaker, and the purified protein solution was extruded to confirm the formation of a filamentous matter in the coagulation liquid (Figure 10A). The filamentous matter formed was taken out from the coagulation liquid with tweezers and air-dried (Figures 10A and 10B).

As a result, it was confirmed that in the purified protein solution obtained by the present purification method, the purity of the protein of interest was high enough to be used as it is as a dope solution for spinning without requiring a further purification step. That is, it was found that a dope solution for spinning can be prepared in a very small number of steps, compared to the preparation steps (aggregation, washing, drying and re-dissolution, etc.) of a dope solution for spinning using a conventional recombinant protein.

### [Example 9 Effect of Reducing Gelation by the Present Purification Method]

The method described in International Publication No. WO 2014/103847 (hereinafter referred to as conventional method) is a method wherein a protein of interest is dissolved in an aprotic polar solvent such as DMSO and a solvent such as water is added to remove impurities and to purify. Although this conventional method is an excellent method, the protein of interest is limited to hydrophilic recombinant proteins having a hydropathy index of 0 or less, and gelation may occur depending on the protein.

The protein (PRT 468 (SEQ ID NO:1), HI: -0.59) with which gelation occurs with this conventional method was purified according to the conventional method and the present purification method, and compared.

### (Conventional method)

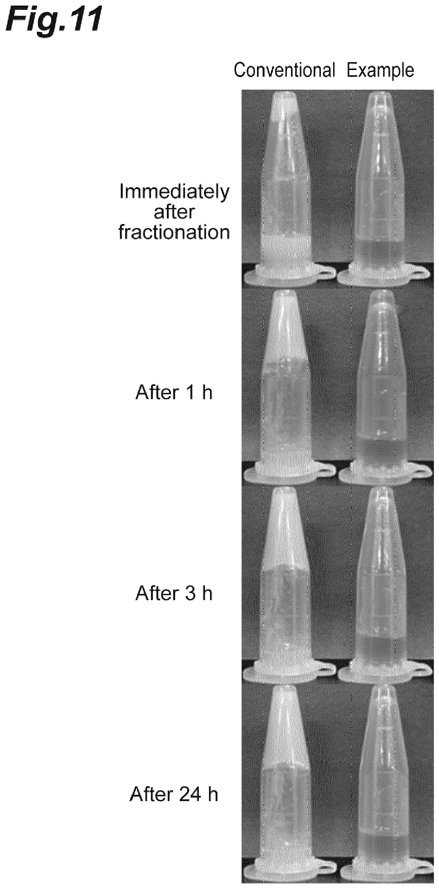

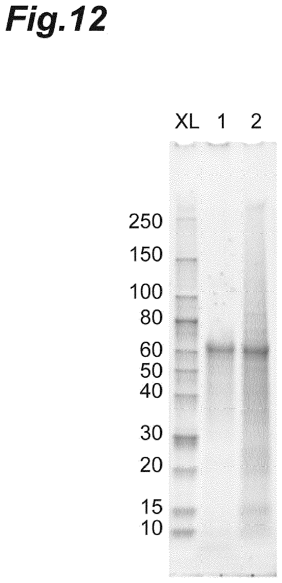

In accordance with the conventional method, 500 µL of DMSO containing 0.5 M lithium chloride was added to 50 mg of dried bacterial cells of Escherichia coli expressing PRT468 and heated to a temperature of 80°C for 30 min to dissolve PRT468. After cooling to room temperature, 500 µL of RO water was added and stirred. A part of the solution was fractionated into Eppendorf tubes, turned upside down immediately after fractionation, after 1 h, after 3 h and after 24 h, and the state of gelation was observed. The results are shown in Figure 11 ("Conventional" on the left). In the conventional method, the purified PRT468 gelled after 1 h. The results of analyzing the solution containing PRT468 before gelation by SDS-PAGE are shown in Lane 1 of Figure 12. Lane XL is the lane where a molecular weight marker protein has migrated.

### (The present purification method)

According to the method described in Example 2, the host cell-derived protein was removed as follows. 1 mL of a first aprotic polar solvent in which lithium chloride was added to be 0.5 M in a solvent mixed with the ratio DMSO:cyclohexanone = 1:1 was added to 50 g of dried bacterial cells of transformed Escherichia coli expressing PRT468, and treated at a temperature of 60°C for 30 min. Centrifugation was performed under the conditions of 11,000 × g and 5 min. The supernatant fraction (first soluble fraction) was removed and the precipitate fraction (first insoluble fraction) was washed with the first aprotic polar solvent at 60°C.

500 µL of DMSO solvent containing 0.5 M lithium chloride (second aprotic polar solvent) was added to the washed precipitate fraction, and was treated at 80°C for 30 min to dissolve PRT468. A part of the solution was fractionated into Eppendorf tubes, turned upside down immediately after fractionation, after 1 h, after 3 h and after 24 h, and the state of gelation was observed. The results are shown in Figure 11 ("Present Example" on the right). According to the present purification method, gelation did not occur even after 24 h.

The solution was centrifuged under the conditions of 11,000 × g and 5 min and the precipitate fraction (second insoluble fraction) was removed. 700 µL of isopropanol (poor solvent) was added to 350 µL of the supernatant fraction (second water-soluble fraction) and mixed by inversion for 1 h to aggregate PRT468. Centrifugation was performed under the conditions of 11,000 × g and 5 min and the precipitate fraction (aggregate) was washed with RO water. The washed aggregate was freeze-dried and the resulting powder was analyzed by SDS-PAGE. The results are shown in Lane 2 of Figure 12.

The present purification method and the conventional method have both in common that an aprotic polar solvent is used. However, according to the present purification method, it has been shown that even with a recombinant protein that causes gelation in the conventional method, it can be obtained with almost the same purity as the conventional method without causing gelation.

## Claims

1. A method for purifying a recombinant protein of interest from a recombinant cell expressing intracellularly the recombinant protein as an insoluble matter,
comprising treating the recombinant cell under conditions where a protein derived from a host cell dissolves in a first aprotic polar solvent to which an inorganic salt is added or not but the recombinant protein does not dissolve, removing a first soluble fraction, and then treating the resulting first insoluble fraction under conditions where the recombinant protein dissolves in a second aprotic polar solvent to which an inorganic salt is added.

2. The method for purifying a recombinant protein according to claim 1, further comprising, after the treatment under conditions where the recombinant protein dissolves in the second aprotic polar solvent to which an inorganic salt is added, removing a second insoluble fraction to obtain a second soluble fraction containing the recombinant protein.

3. The method for purifying a recombinant protein according to claim 2, further comprising treating the second soluble fraction obtained by removing the second insoluble fraction using a poor solvent for the recombinant protein, thereby aggregating the recombinant protein to obtain the purified recombinant protein as an aggregate.

4. A method for purifying a recombinant protein, comprising the following steps (A) to (D):
(A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
(B) a step of removing a first soluble fraction and recovering a first insoluble fraction comprising the recombinant protein;
(C) a step of treating the recovered first insoluble fraction using a second aprotic polar solvent to which an inorganic salt is added, under conditions where the recombinant protein dissolves; and
(D) a step of removing a second insoluble fraction to obtain a second soluble fraction comprising the recombinant protein.

5. The method for purifying a recombinant protein according to claim 4, further comprising:
(E) a step of treating the second soluble fraction using a poor solvent for the recombinant protein, thereby aggregating the recombinant protein and recovering the purified recombinant protein as an aggregate.

6. The method for purifying a recombinant protein according to any one of claims 1 to 5, wherein the conditions are a temperature condition.

7. The method for purifying a recombinant protein according to any one of claims 1 to 6, wherein the first or second aprotic polar solvent has a dipole moment of 3.0 D or more.

8. The method for purifying a recombinant protein according to any one of claims 1 to 7, wherein the inorganic salt is selected from the group consisting of alkali metal halides, alkaline earth metal halides, alkaline earth metal nitrates and thiocyanates.

9. The method for purifying a recombinant protein according to any one of claims 1 to 8, wherein the recombinant protein is a structural protein.

10. The method according to claim 9, wherein the structural protein is derived from a protein selected from the group consisting of collagen, elastin, resilin, silkworm silk and spider silk.

11. A method for producing an artificial polypeptide fiber, comprising the following steps (A) to (D) and (F):
(A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
(B) a step of removing a first soluble fraction and recovering a first insoluble fraction comprising the recombinant protein;
(C) a step of treating the recovered first insoluble fraction using a second aprotic polar solvent to which an inorganic salt is added, under conditions where the recombinant protein dissolves;
(D) a step of removing a second insoluble fraction to obtain a second soluble fraction comprising the recombinant protein; and
(F) a step of applying the second soluble fraction as a filamentous liquid to a coagulation liquid, thereby coagulating the recombinant protein in filament form, and recovering the recombinant protein to obtain an undrawn yarn.

12. A method for producing a polypeptide film, comprising the following steps (A) to (D) and (G):
(A) a step of treating a recombinant cell expressing intracellularly a recombinant protein of interest as an insoluble matter using a first aprotic polar solvent to which an inorganic salt is added or not, under conditions where a protein derived from a host cell dissolves but the recombinant protein does not dissolve;
(B) a step of removing a first soluble fraction and recovering a first insoluble fraction comprising the recombinant protein;
(C) a step of treating the recovered first insoluble fraction using a second aprotic polar solvent to which an inorganic salt is added, under conditions where the recombinant protein dissolves;
(D) a step of removing a second insoluble fraction to obtain a second soluble fraction comprising the recombinant protein; and
(G) a step of forming a coating film of a predetermined thickness on a flat plate resistant to the second aprotic polar solvent using the second soluble fraction, and removing the second aprotic polar solvent from the coating film to obtain a polypeptide film.
